# EUROPEAN PATENT SPECIFICATION

(11) **EP 3 897 809 B1**
(45) Date of publication and mention of the grant of the patent: **13.09.2023**
(21) Application number: 19842895.5
(22) Date of filing: 18.12.2019
(51) Int. Cl.: A61M 37/00, A61B 5/15

(54) **KIT AND COSMETIC PROCESS USING MICRONEEDLE SHEET**
KIT UND KOSMETISCHES VERFAHREN UNTER VERWENDUNG VON MIKRONADELFOLIE
KIT ET PROCÉDÉ COSMÉTIQUE UTILISANT UNE FEUILLE À MICRO-AIGUILLES

(30) Priority: 21.12.2018 JP 2018239583
(43) Date of publication of application: 27.10.2021
(73) Proprietor: L'OREAL, 75008 Paris (FR); Liu, Shu, Kawasaki-shi, Kanagawa 213-0012 (JP); Agarwal, Gaurav, Kawasaki-shi, Kanagawa, 213-0012 (JP)
(72) Inventor: LIU, Shu, Kawasaki-shi Kanagawa 2130012 (JP); AGARWAL, Gaurav, Kawasaki-shi Kanagawa 2130012 (JP)
(74) Representative: Cabinet Nony
(86) International application number: PCT/JP2019/051644
(87) International publication number: WO 2020/130157

(56) References cited:
- EP-A1- 3 111 987
- WO-A1-2015/183179
- WO-A1-2017/151745
- WO-A1-2017/151806
- WO-A1-2017/209774
- WO-A2-2008/091602
- US-A- 5 487 726
- US-A1- 2014 276 378
- US-A1- 2017 296 465

## Description

### TECHNICAL FIELD

The present invention relates to a kit comprising at least one microneedle sheet comprising a plurality of microneedles, as well as a cosmetic process for a keratin substance such as the skin and the lips, using the microneedle sheet.

### BACKGROUND ART

The stratum corneum (SC) constitutes the main barrier to exogenous substances including small molecular weight materials. In general, exogenous substances permeating the skin must diffuse through the highly organized intercellular lipid bilayers of the stratum corneum. This intercellular microroute, which is lipophilic, is the primary pathway for exogenous substances to pass through the SC barrier by passive diffusion along a concentration gradient between a delivery vehicle and the SC. It is difficult for some exogenous substances to penetrate into the skin.

In order to provide exogenous substances deeper into the skin, it is possible to perform injections using a conventional needle. However, such injections cause pain, and need to be performed by a professional such as a doctor. Thus, injections using a conventional needle are not common for cosmetic purposes.

The concept of using a micro-structured device with a plurality of microneedles to breach the stratum corneum (SC) barrier was first proposed in the 1970s. The production of microneedle arrays has been described in the art, for example in WO 2008/139786, WO 2009/040548, WO 2015/147040 and WO 2016/076442. Microneedles have an advantage of potentially penetrating the SC without pain caused by the use of conventional needles for injections, and can be self-administered.

In general, microneedles are arrayed on a substrate layer to form a microneedle sheet. A microneedle sheet is applied on a keratin substance such as the skin for some time so that the microneedles can be inserted into the keratin substance and dissolved or disintegrated in the keratin substance by the moisture or water inside the keratin substance.

### DISCLOSURE OF INVENTION

There are some conventional devices for applying microneedles onto the skin. One of such devices is an applicator with a roller on which microneedles are formed.

However, as microneedles are formed onto the roller, storing the applicator without breaking the microneedles is difficult for such a conventional device.

An objective of the present invention is to provide a kit which can allow a user to easily store an applicator for microneedles without breaking the microneedles, as well as a cosmetic process which can use the kit.

The above objective can be achieved by a kit comprising:
at least one microneedle sheet comprising a substrate layer and a plurality of microneedles on the substrate layer, the microneedles comprising at least one water-soluble or water-dispersible polymer;
   and
at least one applicator comprising at least one application surface, wherein the application surface of the applicator is curved,
   wherein
the substrate layer of the microneedle sheet is capable of being attached to the application surface of the applicator.

It is preferable that the substrate layer of the microneedle sheet be detachable from the application surface of the applicator, after being attached to the application surface of the applicator.

The microneedle sheet may comprise at least one base layer which may be fixed onto the substrate layer.

The microneedle sheet may comprise at least one adhesive layer which may be fixed onto the substrate layer or the base layer, if present.

The microneedle sheet may comprise at least one releasing layer which may be placed on the adhesive layer.

The microneedle may have a height of from 50 to 1000 microns, preferably from 100 to 750 microns, and more preferably from 150 to 500 microns.

The application surface of the applicator is curved. The applicator may comprise at least one cylindrical roller having a curved side surface, and the curved side surface of the cylindrical roller may comprise the application surface.

The kit according to the present invention may further comprise at least one container wherein the container is capable of storing the microneedle sheet.

The container may comprise at least one soft and/or porous article, preferably selected from sponges, woven or non-woven fabrics and combinations thereof.

The kit according to the present invention may further comprise at least one cosmetic composition.

The present invention also relates to a cosmetic process for a keratin substance such as the skin or the lips, comprising the steps of:
attaching at least one microneedle sheet to at least one application surface of at least one applicator, wherein the application surface of the applicator is curved,
   wherein
   the microneedle sheet comprises a substrate layer and a plurality of microneedles on the substrate layer, the microneedles comprising at least one water-soluble or water-dispersible polymer,
by attaching the substrate layer to the application surface of the applicator;
   and
applying the applicator onto the keratin substance such that the microneedles contact the keratin substance.

### BRIEF DESCRIPTION OF DRAWINGS

Figure 1 shows a cross-sectional view of an example of a microneedle sheet to be used for the present invention.
Figure 2 shows a perspective view of an example of an applicator comprising at least one cylindrical roller having a curved side surface, wherein the curved side surface of the cylindrical roller comprises the application surface.
Figure 3 shows a perspective view of an example of an applicator to which the substrate layer of a microneedle sheet is attached.
Figure 4 shows a perspective view of how to use an applicator with a microneedle sheet.
Figure 5 shows a front view of an example of an applicator (not claimed) comprising at least one stamp having at least one flat surface, wherein the flat surface comprises the application surface.
Figure 6 shows a cross-sectional view of an example of a container which may be used for the present invention.

### BEST MODE FOR CARRYING OUT THE INVENTION

After diligent research, the inventors have discovered that it is possible to provide a kit which can allow a user to easily store an applicator for microneedles without breaking the microneedles.

Thus, one aspect of the present invention is a kit comprising:
at least one microneedle sheet comprising a substrate layer and a plurality of microneedles on the substrate layer, the microneedles comprising at least one water-soluble or water-dispersible polymer;
   and
at least one applicator comprising at least one application surface, wherein the application surface of the applicator is curved,
   wherein
the substrate layer of the microneedle sheet is capable of being attached to the application surface of the applicator.

According to the present invention, the microneedle sheet is separated from the applicator when not being used. The microneedle sheet can be attached to the applicator when being used. Therefore, a user can easily store the applicator without breaking the microneedles.

It is preferable that the substrate layer of the microneedle sheet be detachable from the application surface of the applicator after being attached to the application surface of the applicator. In this case, the applicator can be repeatedly used by exchanging the microneedle sheet on the applicator. The exchange of the microneedle sheet can be performed by a user.

If the kit according to the present invention further comprises at least one container wherein the container is capable of storing the microneedle sheet, storing the microneedle sheet without breaking the microneedles can be performed more easily.

Since microneedles do not cause any pain, the present invention can provide cosmetic treatments without pain. Thus, the kit according to the present invention is convenient for cosmetic or non-therapeutic treatments.

Hereafter, the kit and cosmetic process according to the present invention will be described in a detailed manner.

### [Kit]

The kit according to the present invention comprises:
at least one microneedle sheet comprising a substrate layer and a plurality of microneedles on the substrate layer, the microneedles comprising at least one water-soluble or water-dispersible polymer;
   and
at least one applicator comprising at least one application surface, wherein the application surface of the applicator is curved,
   wherein
the substrate layer of the microneedle sheet is capable of being attached to the application surface of the applicator.

### {Microneedle Sheet}

The microneedle sheet to be used for the kit according to the present invention comprises a substrate layer and a plurality of microneedles on the substrate layer, wherein the microneedles comprise at least one water-soluble or water-dispersible polymer.

The microneedle sheet to be used for the present invention may be a cosmetic device, preferably a cosmetic device for a keratin substance, and more preferably a cosmetic device for the skin, in particular the skin of the face, as well as the lips.

### (Microneedles)

The microneedle sheet to be used for the present invention comprises a plurality of microneedles.

The microneedles are present on the surface of a substrate layer. The microneedles may be present on 50% or more, preferably 70% or more, and more preferably 90% or more of the surface of the substrate layer.

It is preferable that the microneedles be present on one of the surfaces of the substrate layer.

It is preferable that the microneedles of the microneedle sheet to be used for the present invention be designed to penetrate or enter into the stratum corneum of the skin, in particular the skin of the face, as well as the lips.

A microneedle can be any suitable size and shape to puncture the stratum corneum. It may be preferable that the microneedles be designed to pierce and cross the stratum corneum. The microneedles may be capable of creating openings in the stratum corneum.

If necessary, the height of the microneedles may be altered so as to allow penetration into the epidermis and/or dermis of the skin, preferably into the upper dermis, and more preferably into the lower dermis.

The shape of the microneedles is not limited as long as the shape is a "needle". It will be apparent to those skilled in the art that the microneedles for the present invention can take any reasonable shape, including, but not limited to, cones, rods and/or pillars. As such, the microneedles may have the same diameter at the tip as at the base or may taper in diameter in the direction from the base to the tip.

For example, the shape of the microneedle may be in the form of a triangular pyramid, a square pyramid or a pentagonal pyramid. Alternatively, the microneedle may be in the form of a cylinder preferably with a tip which may be formed by diagonally cutting the cylinder.

The cross section of the microneedle may take any geometric form including, circular, triangular, square, rectangular, polyhedral, regular or irregular forms, and the like. In an embodiment, a group of microneedles may take the form of hollow microcapillaries.

Thus, reference is made to "microneedles" as a type of microprotrusion or microprojection which is being employed. It will be understood by persons skilled in the art that in many cases the same inventive principles apply to the use of other microprotrusions or microprojections to penetrate the skin. Other microprotrusions or microprojections may include, for example, microblades as described in U.S. Patent No. 6,219,574 and Canadian Patent Application No. 2,226,718, and edged microneedles as described in U.S. Patent No. 6,652,478.

The height or length of the microneedle of the microneedle sheet to be used for the present invention may be from 50 to 1000 microns, preferably from 100 to 750 microns, and more preferably from 150 to 500 microns.

According to one embodiment of the present invention, the microneedle is in the form of a cone.

The cone may comprise a distal end such as a tip and a base. The shape of the base may be a circle or oval.

The height or length of the cone of the microneedle of the microneedle sheet to be used for the present invention may be from 50 to 1000 microns, preferably from 100 to 750 microns, and more preferably from 150 to 500 microns.

The base of the cone of the microneedle of the microneedle sheet to be used for the present invention may have a diameter or width of from 50 to 350 microns, preferably from 100 to 300 microns, and more preferably from 150 to 250 microns. If the base of the cone of the microneedle of the microneedle sheet to be used for the present invention is in the shape of an oval or ellipse, the length of the major axis or width of the oval may be from 50 to 350 microns, preferably from 100 to 300 microns, and more preferably from 150 to 250 microns.

The microneedle may have an aspect ratio (length/width at base) of at least about 3:1, at least about 2:1, or at least about 1:1. The ratio of (the height of the cone)/(the diameter of the base of the cone) of the microneedle may be 1 or more, preferably 1.5 or more, and more preferably 2.0 or more.

Preferably, the microneedles do not fracture by force when a pressure of insertion of less than 50.0 N/cm², for example less than 20.0 N/cm², such as less than 10 N/cm² is exerted on the microneedles along their length.

It is also preferable that the microneedle sheet to be used for the present invention, in particular the microneedles of the microneedle sheet, have a Young modulus of 50 N/mm or more, preferably 55 N/mm or more, and more preferably 60 N/mm or more.

It may be preferable that the microneedle be capable of penetrating into a keratin substance, such as the skin or the lips, to a depth of 200 microns or less, preferably 180 microns or less, and more preferably 160 microns or less.

The microneedle can be dissolvable or non-dissolvable. It is preferable that the microneedle be dissolvable.

By "dissolvable microneedle" is meant that the microneedle can be broken down or disintegrated inside a keratin substance such as the skin or the lips by, for example, a natural moisturizing factor or external moisture.

The microneedles of the microneedle sheet to be used for the present invention may comprise at least one water-soluble or water-dispersible polymer. Here, the terms "water-soluble" and "water-dispersible" mean soluble and dispersible, respectively, when in contact with water. A single water-soluble or water-dispersible polymer may be used. Two or more water-soluble or water-dispersible polymers may be used in combination.

It is preferable that the water-soluble or water-dispersible polymer be soluble or dispersible in the skin or the lips. Thus, in one embodiment of the present invention, the water-soluble or water-dispersible polymer is capable of being dissolved or dispersed after insertion into a keratin substance such as the skin or the lips. Due to the solubility or dispersibility of the polymer, the microneedles of the microneedle sheet to be used for the present invention can effectively release an agent or agents, if present, in the microneedles. Optional external water combined with the application of the microneedle sheet can be used to accelerate the dissolution or dispersion of the microneedles.

It is preferable that the water-soluble or water-dispersible polymer be dissolvable in the surface layer of a keratin substance such as the skin or the lips.

The water-soluble or water-dispersible polymer may be selected from hyaluronic acids (in particular lower molecular weight hyaluronic acid), monosaccharides, disaccharides, oligosaccharides, polysaccharides (including derivatives thereof such as hydroxymethylcellulose), dextrins, dextrans, polyethylene glycols, polyvinyl alcohols, poly(methylvinylether/maleic anhydride), polyvinylpyrrolidone, poly(methyl/vinyl ether/maleic acid) (PMVE/MA) and esters thereof, poly(methyl/vinyl ether/maleic anhydride) (PMVE/MAH), and mixtures thereof.

The water-soluble or water-dispersible polymer may have a molecular weight of from 10,000 to 200,000 Dalton, preferably from 30,000 to 150,000 Dalton, and more preferably from 50,000 to 100,000 Dalton.

The low molecular weight hyaluronic acid may have a molecular weight of 100 kDa or less, preferably 70 kDa or less, and more preferably 50 kDa or less.

The polyvinylpyrrolidone may have a molecular weight between 1 kDa and 300 kDa, preferably between 5 kDa and 200 kDa, and more preferably between 7 kDa and 100 kDa.

The poly(methyl/vinyl ether/maleic acid) (PMVE/MA) and esters thereof, and poly(methyl/vinyl ether/maleic anhydride) (PMVE/MAH) are known as Gantrez-type polymers.

The amount (solid basis) of the water-soluble or water-dispersible polymer(s) in the microneedle of the microneedle sheet to be used for the present invention may be 50% by weight or more, preferably 60% by weight or more, and more preferably 70% by weight or more, relative to the total weight of the microneedle. The amount (solid basis) of the water-soluble or water-dispersible polymer(s) in the microneedle of the microneedle sheet to be used for the present invention may be 100% by weight or less, preferably 90% by weight or less, and more preferably 80% by weight or less, relative to the total weight of the microneedle. Thus, the amount (solid basis) of the water-soluble or water-dispersible polymer(s) in the microneedle of the microneedle sheet to be used for the present invention may be from 50% to 100% by weight, preferably from 60% to 90% by weight, and more preferably from 70% to 80% by weight, relative to the total weight of the microneedle.

It may be possible that the microneedle of the microneedle sheet to be used for the present invention comprise at least one material which is swellable, more preferably water-swellable, and even more preferably swellable in a keratin substance such as the skin or the lips. The above material may be a polymer which is swellable, more preferably water-swellable, and even more preferably swellable in the keratin substance. Here, the term "water-swellable" means swellable when in contact with water. The above swellable material or polymer may have a high swellability such that it can swell to at least over 10 times in a 1-hour *in vitro* incubation in a physiological saline solution or phosphate buffered saline, preferably at least 20 times in 1-hour incubation, more preferably at least 30 times in 1-hour incubation, even more preferably at least 40 times in 1-hour incubation, and most preferably about 45-55 times in 1-hour incubation.

It may be preferable that at least the distal end portion of the microneedle swell upon insertion into the keratin substance, more preferably within less than 1 hour, and even more preferably to at least 2 times within 24 hours after insertion into the keratin substance.

The above swellable material, preferably the above swellable polymer, may have a high viscoelasticity such that it can form a gel after the *in vitro* incubation in a physiological saline solution or phosphate buffered saline. The gel exhibits a high elastic modulus G', a high viscous modulus G", a Tangent (δ) (Tangent (δ) = G"/G') of less than 1, and a high consistency G* (G^{∗2}=G'² + G"²) even at low frequency (0.01 Hz) in a dynamic frequency sweep test with a Rheometer.

In one embodiment, the above swellable material, preferably the swellable polymer, is not water-soluble or not water-dispersible.

In another embodiment, the above swellable material, preferably the swellable polymer, may be a hydrogel-forming polymer.

The above swellable polymer may be selected from high molecular weight hyaluronic acids, cross-linked hyaluronic acids, cross-linked polyethylene glycol, polyethylene glycol cross-linked poly-lactic acid or poly-glycolic acid or poly-lactic-co-glycolic acid or poly dioxanone, poly(styrene)-block-poly(acrylic acid), polyethylene glycol cross-linked PMVE/MA, cross-linked polyvinylpyrrolidone, sodium starch glycolate; cellulose; natural and synthetic gums; alginates; sodium polyacrylate PEG-crosslinked poly(methyl/vinyl ether/maleic acid) (PMVE/MA) and esters thereof, PEG-crosslinked poly(methyl/vinyl ether/maleic anhydride) (PMVE/MAH) and esters thereof, and mixtures thereof.

The high molecular weight hyaluronic acid may have a molecular weight of more than 500 kDa, preferably more than 1000 kDa, and more preferably more than 2100 kDa, and preferably less than 10000 kDa.

Unless otherwise defined, the molecular weight here means a number average molecular weight.

The poly(methyl/vinyl ether/maleic acid) (PMVE/MA) and esters thereof, and poly(methyl/vinyl ether/maleic anhydride) (PMVE/MAH) are known as Gantrez-type polymers.

The amount (solid basis) of the swellable material(s) in the microneedle of the microneedle sheet to be used for the present invention may be 1% by weight or more, preferably 5% by weight or more, and more preferably 10% by weight or more, relative to the total weight of the microneedle. The amount (solid basis) of the swellable material(s) in the microneedle of the microneedle sheet to be used for the present invention may be 30% by weight or less, preferably 25% by weight or less, and more preferably 20% by weight or less, relative to the total weight of the microneedle. Thus, the amount (solid basis) of the swellable material(s) in the microneedle of the microneedle sheet to be used for the present invention may be from 1% to 30% by weight, preferably from 5% to 25% by weight, and more preferably from 10% to 20% by weight, relative to the total weight of the microneedle.

If the microneedle of the microneedle sheet to be used for the present invention comprises at least one swellable material, preferably at least one swellable polymer, the microneedle may be swellable such that it can improve the aesthetic appearance of a keratin substance, preferably the skin, and more preferably the skin of the face, by reducing the appearance of wrinkles.

In other words, if the microneedle is swellable, it can swell in the skin to further increase the volume of the microneedle along with its absorption of, for example, water in the skin. Such volume expansion beneath the skin surface of a wrinkle site can effectively push the wrinkles from inside the skin and makes the wrinkles become shallower and wider. Thus, the wrinkles can be reduced or made less noticeable.

The apical separation distance between each of the individual microneedles on a substrate layer can be modified to ensure the penetration of the skin or the lips by the microneedles while having a sufficiently small separation distance to provide high transdermal transport rates.

In one embodiment, the range of apical separation distances between microneedles can be in the range of 10-1000 µm, such as 30-800 µm or 50-600 µm. This may allow a compromise to be achieved between efficient penetration of the stratum corneum by as many microneedles as possible and the necessary margin for possible swelling of the microneedles if they are swellable.

In one embodiment, the density of microneedles may be from 100 to 2000 microneedles/cm², preferably 200 to 1000 microneedles/cm², and even more preferably 200 to 500 microneedles/cm².

### (Polyol)

According to one embodiment, at least one of the microneedles of the microneedle sheet may comprise at least one polyol. A single type of polyol may be used. Two or more polyols may be used in combination.

It is preferable that the polyol be in the form of a liquid at ambient temperature such as 25°C under atmospheric pressure (760 mmHg or 10⁵ Pa).

The term "polyol" here means an alcohol having two or more hydroxy groups, and does not encompass a saccharide or a derivative thereof. The derivative of a saccharide includes a sugar alcohol which is obtained by reducing one or more carbonyl groups of a saccharide, as well as a saccharide or a sugar alcohol in which the hydrogen atom or atoms in one or more hydroxy groups thereof has or have been replaced with at least one substituent such as an alkyl group, a hydroxyalkyl group, an alkoxy group, an acylgroup or a carbonyl group.

Polyols used in the present invention are liquid at ambient temperature such as 25°C under atmospheric pressure (760 mmHg or 10⁵ Pa).

The polyol may be a C₂-C₂₄ polyol, preferably a C₂-C₉ polyol, comprising at least 2 hydroxy groups, and preferably 2 to 5 hydroxy groups.

The polyol may be a natural or synthetic polyol. The polyol may have a linear, branched or cyclic molecular structure.

The polyol may be selected from glycerins and derivatives thereof, and glycols and derivatives thereof. The polyol may be selected from the group consisting of glycerin, diglycerin, polyglycerin, ethyleneglycol, diethyleneglycol, propyleneglycol, dipropyleneglycol, butyleneglycol, pentyleneglycol, hexyleneglycol, C₆-C₂₄ polyethyleneglycol, 1,3-propanediol, 1,4-butanediol, and 1,5-pentanediol.

If the microneedle(s) of the microneedle sheet include(s) polyol(s), the brittleness of the microneedle(s) can be reduced or controlled.

### (Saccharide)

According to one embodiment, at least one of the microneedles of the microneedle sheet may comprise at least one saccharide or derivative thereof. A single type of saccharide or derivative thereof may be used. Two or more saccharides, derivatives thereof, or a combination of saccharide(s) and derivative(s) thereof may be used.

The term "saccharide" here means a polyalcohol composed of one or more glucidic units, and may have at least one carbonyl group. The derivative of a saccharide includes a sugar alcohol which is obtained by reducing one or more carbonyl groups of a saccharide, as well as a saccharide or a sugar alcohol in which the hydrogen atom or atoms in one or more hydroxy groups thereof has or have been replaced with at least one substituent such as an alkyl group, a hydroxyalkyl group, an alkoxy group, an acylgroup or a carbonyl group.

The saccharide may be selected from the group consisting of monosaccharides, oligosaccharides, polysaccharides, and mixtures thereof.

The monosaccharide may be selected from the group consisting of glucose, fructose, galactose, mannose, talose, sorbose, xylose, lyxose, fucose, arabinose, rhamnose, ribose, ribulose, xylulose, sorbitol, triose, pentose, hexose, ketose, glucosamine, and mixtures thereof.

The oligosaccharide may be selected from the group consisting of maltose, lactose, saccharose, cellobiose, trehalose, sucrose, flucto-oligosaccharide, dextrin, and mixtures thereof. The preferred oligosaccharides may be composed of 2 to 10 monosaccharides.

The polysaccharide may be selected from alginic acid, guar gum, xanthan gum, gum arabic, arabinogalactan, carrageenan, agar, karaya gum, gum tragacanth, tara gum, pectin, locust bean gum, cardolan, jellan gum, dextran, pullulan, hyaluronic acid, cellulose and its derivatives, and mixtures thereof.

The derivative of a saccharide may be selected from the group consisting of erythritol, lactitol, maltitol, mannitol, sorbitol and xylitol, in which at least one hydrogen in the hydroxyl groups thereof may have been replaced with at least one substituent such as an alkyl group, a hydroxyalkyl group, an alkoxy group, an acylgroup or a carbonyl group.

The above saccharides can exist in the form of L or D isomers.

### (Cosmetic Active Ingredient)

According to one embodiment, at least one of the microneedles of the microneedle sheet used for the present invention may comprise at least one cosmetic active ingredient. A single cosmetic active ingredient may be used. Two or more cosmetic active ingredients may be used in combination.

The type of the cosmetic active ingredient is not limited. The cosmetic active ingredient may be selected from the group consisting of whitening agents, anti-oxidants, cleansing agents, anti-ageing agents, skin texture treatment agents and combinations thereof.

For example, an anti-aging agent may be used as the cosmetic active ingredient.

As examples of the anti-aging agent, mention may be made of anti-oxidants, moisturizers, free-radical scavengers, keratolytic agents such as alpha or beta-hydroxy acids, vitamins such as vitamin C and derivatives thereof and vitamin E and derivatives thereof, anti-elastase and anti-collagenase agents, protides, fatty acid derivatives, steroids, trace elements, bleaching agents, plant oils such as almond oil, extracts of algae and of planktons, enzymes and coenzymes, flavonoids and ceramides, and mixtures thereof.

The amount of the cosmetic active ingredient(s) in the microneedle of the microneedle sheet used for the present invention is not limited, and may be from 0.01% to 10% by weight, preferably from 0.05% to 5% by weight, and more preferably from 0.1% to 1% by weight, relative to the total weight of the microneedle.

It is also possible that the amount of the cosmetic active ingredient(s) in the microneedle of the microneedle sheet to be used for the present invention be less than 0.01% by weight, relative to the total weight of the microneedle. In one embodiment, the microneedle of the microneedle sheet according to the present invention may include no cosmetic active ingredient.

### (Substrate Layer)

The microneedle sheet to be used for the present invention comprises a substrate layer on which the microneedles are present or placed.

The substrate layer of the microneedle sheet to be used for the present invention may be dissolvable or non-dissolvable.

It is preferable that the substrate layer of the microneedle sheet to be used for the present invention be dissolvable in water or disintegrable in water.

The substrate layer of the microneedle sheet to be used for the present invention may comprise at least one water-soluble or water dispersible polymer, as explained above. In other words, the above explanations for the water-soluble or water-dispersible polymer which may be comprised in the microneedles of the microneedle sheet can apply to the water-soluble or water-dispersible polymer which may be comprised in the substrate layer.

For example, the amount (solid basis) of the water-soluble or water-dispersible polymer(s) in the substrate layer of the microneedle sheet to be used for the present invention may be 50% by weight or more, preferably 60% by weight or more, and more preferably 70% by weight or more, relative to the total weight of the substrate layer. The amount (solid basis) of the water-soluble or water-dispersible polymer(s) in the substrate layer of the microneedle sheet to be used for the present invention may be 100% by weight or less, preferably 90% by weight or less, and more preferably 80% by weight or less, relative to the total weight of the substrate layer. Thus, the amount (solid basis) of the water-soluble or water-dispersible polymer(s) in the substrate layer of the microneedle sheet to be used for the present invention may be from 50% to 100% by weight, preferably from 60% to 90% by weight, and more preferably from 70% to 80% by weight, relative to the total weight of the substrate layer.

The substrate layer and the microneedles may be or may not be integrated.

If the substrate layer and the microneedles are integrated, the substrate layer and the microneedles may comprise at least one common water-soluble or water dispersible polymer.

Thus, in one embodiment, the substrate layer and the microneedles can be a single element comprising at least one common water-soluble or water-dispersible polymer. Preferably, the single element can be prepared by using the same water-soluble or water-dispersible polymer(s).

On the other hand, if the substrate and the microneedles are not integrated, i.e., if the substrate layer is different or distinct from the microneedles, the substrate layer and the microneedles may be made from different materials.

Thus, in another embodiment, the substrate layer and the microneedles can be an assembly of two different or distinct elements.

In the latter embodiment, the substrate layer may be, for example, made from fibers or a polymeric film.

The fibers may be made from natural fibers such as cotton, semi-natural fibers such as viscose, synthetic fibers such as polyester and polypropylene fibers, and combinations thereof. The porous base layer may be selected from woven or non-woven fabrics.

The polymeric film may be made from synthetic resins such as polyurethanes, polystyrenes, polycarbonates, polyethylene terephthalates, poly(ethylene-co-vinyl alcohol), polyethylenes, polyesters, polyamides, and combinations thereof.

The substrate layer may be cut so as to be in the form of a patch, a disc, a mask, a towel, a glove, a precut roll, or any other form suitable for a cosmetic use.

### (Additional Layer)

The microneedle sheet to be used for the present invention may comprise at least one additional layer.

In one embodiment, the microneedle sheet comprises at least one base layer, as the additional layer, and the base layer is fixed onto the substrate layer. For fixing, any means to firmly attach the base layer on the substrate layer may be used. For example, any adhesive agent may be used to fix the base layer on the substrate layer.

The form and material of the base layer are not limited.

The base layer may be a porous or non-porous base layer.

The porous base layer may be composed of fibers. The fibers may be made from natural fibers such as cotton, semi-natural fibers such as viscose, synthetic fibers such as polyester and polypropylene fibers, and combinations thereof. The porous base layer may be selected from woven or non-woven fabrics.

The non-porous base layer may be composed of a polymeric film. The polymeric film may be made from synthetic resins such as polyurethanes, polystyrenes, polycarbonates, polyethylene terephthalates, poly(ethylene-co-vinyl alcohol), polyethylenes, polyesters, polyamides, and combinations thereof.

If the microneedles and the substrate layer of the microneedle sheet are different or distinct elements, the substrate layer may function as the base layer. Therefore, in this case, the base layer may not be necessary.

In one embodiment, the microneedle sheet comprises at least one adhesive layer as the additional layer, and the adhesive layer is fixed onto the substrate layer or the base layer, if present.

The material of the adhesive layer is not limited. It is preferable to use an adhesive layer which can be detached from the application surface of an applicator. For example, it is preferable to use an adhesive layer which is peelable or removable from the application surface of an applicator.

It may be preferable to use a pressure-sensitive adhesive agent to form the adhesive layer. As the pressure-sensitive adhesive agent, any conventional pressure-sensitive adhesive agent can be used. The pressure-sensitive adhesive agent may comprise at least one adhesive polymer. The adhesive polymer may be selected from the group consisting of (meth)acrylate polymers, vinyl acetate-acrylate copolymers, copolymers including polyisobutylene, polystyrene, or polybutadiene, rosin-based resins, polyterpene resins, petroleum-based resins, terpene phenol resins, silicone resins, natural or synthetic rubbers, and mixtures thereof.

In one embodiment, the microneedle sheet comprises at least one releasing layer, as the additional layer, and the releasing layer is placed on the adhesive layer.

The releasing layer can protect the adhesive layer.

The material of the releasing layer is not limited. It is preferable to use a thin film or sheet, such as a paper, which is coated with at least one releasing agent. As the releasing agent, any conventional releasing agent can be used. It is preferable that the releasing layer be easily peelable from the adhesive layer.

It is preferable to form at least one tab for the releasing layer because a user can grasp the tab to easily peel off the releasing layer from the adhesive layer. The tab can be formed, for example, by slightly folding a tip or an edge of the releasing layer.

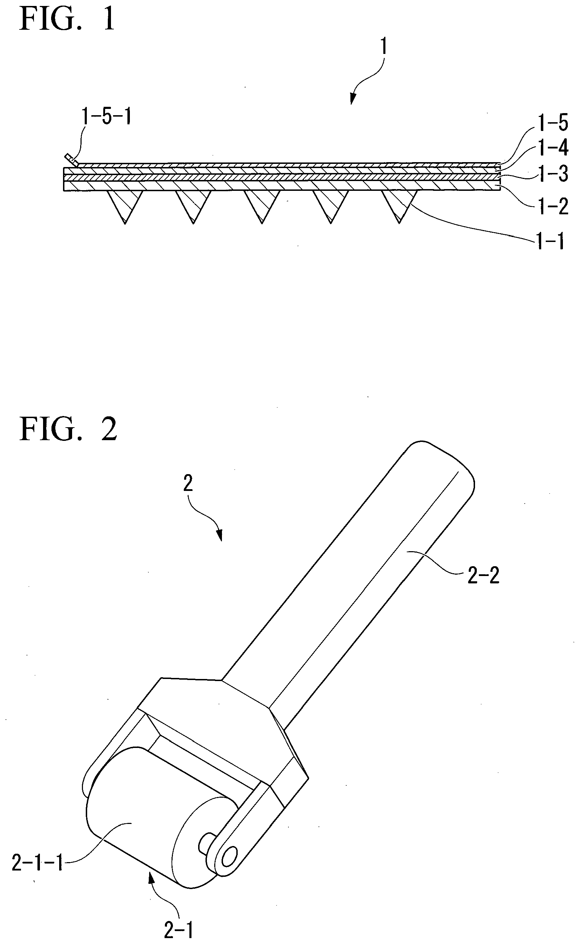

An example of the microneedle sheet to be used for the present invention is illustrated in Figure 1.

Figure 1 shows an example of a multi-layered microneedle sheet 1. The multi-layered microneedle sheet 1 includes microneedles 1-1, a substrate layer 1-2, a base layer 1-3, an adhesive layer 1-4 and a releasing layer 1-5. These layers are stacked in the order shown in Figure 1.

In this example, the substrate layer 1-2 is different from the base layer 1-3. However, if the material(s) of the substrate layer 1-2 are the same as the material(s) of the base layer 1-3, it is possible not to use the base layer 1-3. In this case, the adhesive layer 1-4 can be formed on the substrate layer 1-3.

In this example, the releasing layer 1-5 has a tab 1-5-1. A user can grasp the tab 1-5-1 to peel off the releasing layer 1-5 from the adhesive layer 1-4. Therefore, when the multi-layered microneedle sheet 1 is used, a user can easily peel off the releasing layer 1-5 from the adhesive layer 1-4.

### (Preparation)

There is no limitation regarding how to prepare the microneedle sheet to be used for the present invention. It is possible to prepare the microneedle sheet to be used for the present invention based on conventional technology such as molding, 3D printing and droplet born air blowing.

The microneedle sheet to be used for the present invention can be prepared, for example, by a process comprising the steps of molding a composition comprising at least one water-soluble or water-dispersible polymer, as explained above.

The microneedle sheet to be used for the present invention can be prepared by a process comprising the steps of
(a) providing a mold with cavities corresponding to a negative of the microneedles,
(b) filling a composition comprising at least one water-soluble or water-dispersible polymer, as explained above, into the cavities,
(c) solidifying the composition, for example, by drying at room temperature (and optionally heating) for a period of time such as several hours to form the microneedle sheet, and
(d) removing the microneedle sheet from the mold.

The mold may be made from organic materials such as polyamides and silicones and/or inorganic materials such as aluminum and iron.

At least one evaporable liquid ingredient may be included in the above composition, if necessary, in order to enhance the fluidity of the composition. Examples of the evaporable liquid ingredient are not limited, but may preferably be water and alcohol such as ethanol.

The amount of the evaporable liquid ingredient(s) may be 10% by weight or more, preferably 20% by weight or more, and more preferably 30% by weight or more, relative to the total weight of the composition. The amount of the evaporable liquid ingredient(s) may be 98% by weight or less, preferably 95% by weight or less, and more preferably 90% by weight or less, relative to the total weight of the composition. Thus, the amount of the evaporable liquid ingredient(s) may be from 10% to 98% by weight, preferably from 20% to 95% by weight, and more preferably from 30% to 90% by weight, relative to the total weight of the composition.

The amount of the water-soluble or water-dispersible polymer(s) in the above composition, which is preferably capable of flowing, and more preferably is in the form of a liquid, may be from 2% to 90% by weight, preferably from 5% to 50% by weight, and more preferably from 5% to 30% by weight relative to the total weight of the composition.

If necessary, the above composition may include at least one additional polymer such as the above-explained swellable polymer and/or at least one polyol and/or at least one cosmetic active ingredient, as explained above.

The microneedles and the substrate layer can be prepared simultaneously by the above molding process. In this case, it is preferable that the mold also have a cavity for the substrate layer and the cavities for the microneedles in the mold are connected to the cavity for the substrate layer. Thus, a substrate layer and microneedles can be prepared as a single element.

If the microneedles and the substrate layer of the microneedle sheet are different or distinct elements, the microneedles may be prepared by the above molding process and fixed onto the substrate layer which is separately provided.

The shape of the microneedle sheet to be used for the present invention is not limited, and it may be any shape such as the shape of the lips or a shape suitable for application on the face, depending on the application target of the microneedle sheet.

### {Applicator}

The kit according to the present invention comprises at least one applicator.

The applicator comprises at least one application surface. The application surface is curved. The application surface of the applicator can be applied to a keratin substance such as the skin, in particular the skin of the face, as well as the lips. The application surface of the applicator can contact the keratin substance.

The substrate layer of the microneedle sheet is capable of being attached to the application surface of the applicator. Accordingly, the microneedle sheet is capable of being attached to the application surface.

The means to attach the substrate layer of the microneedle sheet to the application surface of the applicator is not limited. For example, the application surface of the applicator may have a convex and the substrate layer of the microneedle may be inserted to the convex.

It is preferable that the substrate layer of the microneedle sheet be attachable to the application surface of an applicator with an adhesive agent, more preferably an adhesive agent which is peelable or removable from the application surface, and even more preferably a pressure-sensitive adhesive agent.

It is preferable that the microneedle sheet comprise at least one adhesive layer, and the adhesive layer of the microneedle sheet is attached to the application surface of the applicator.

For example, with regard to the example shown in Figure 1, when the microneedle sheet 1 is used, the releasing layer 1-5 is peeled off from the surface of the adhesive layer 1-4, and the adhesive layer 1-4 is attached to the application surface of an applicator.

The type of the applicator is not limited as long as the applicator comprises at least one application surface wherein the application surface is curved.

The application surface is curved, the applicator may comprise at least one cylindrical roller having a curved side surface, wherein the curved side surface of the cylindrical roller comprises the application surface.

Figure 2 shows an embodiment of the invention. The applicator 2 shown in Figure 2 has a roller 2-1 with a curved side surface 2-1-1, and the curved side surface 2-1-1 of the roller 2-1 comprises an application surface to which the substrate layer of a microneedle sheet can be attached.

The applicator 2 also has a handle 2-2. A user of the applicator 2 can grasp the handle 2-2 to use the applicator 2.

When a microneedle sheet is used, a substrate layer of the microneedle sheet is attached to the application surface on the curved side surface 2-1-1 of the applicator 2.

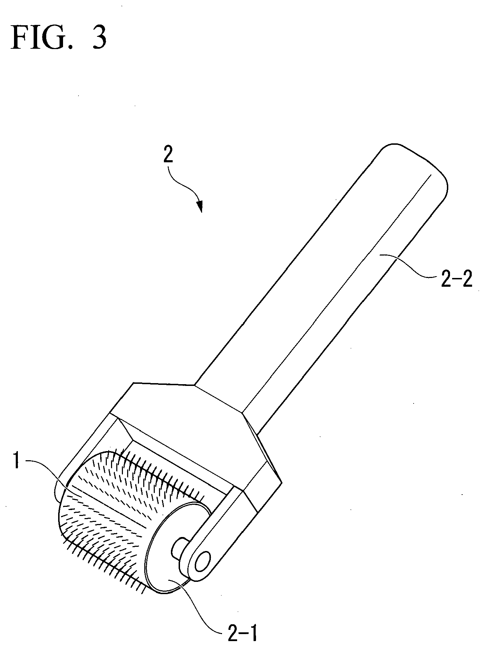

Figure 3 shows the applicator 2 to which the substrate layer of a microneedle sheet 1 is attached. The microneedle sheet 1 is attached to the application surface on the curved side surface of the roller 2-1 such that the substrate layer of the microneedle sheet 1 contacts the application surface. Thus, the microneedles of the microneedle sheet 1 can stand up on the curved side surface of the roller 2.

At least a part of the curved side surface of the roller 2-1 may be covered by the microneedle sheet 1. It is preferable that the entirety of the curved side surface of the roller 2-1 be covered by the microneedle sheet 1.

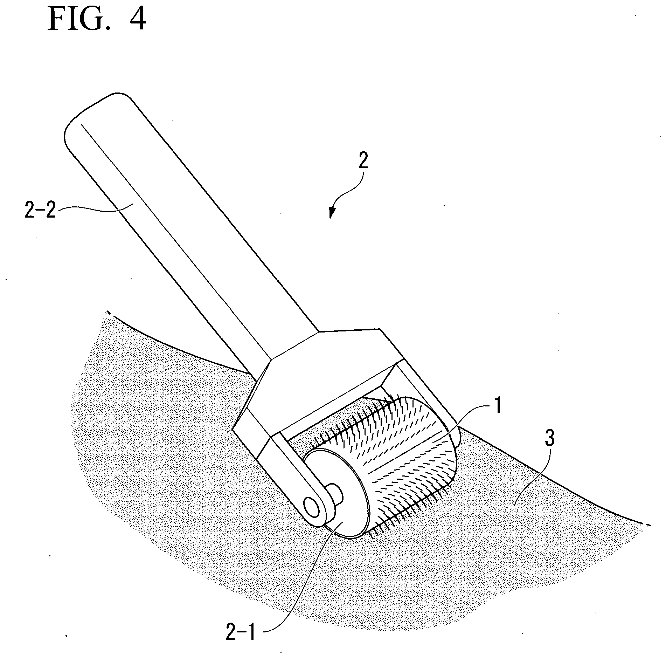

Figure 4 shows how to use the applicator 2 with the microneedle sheet 1.

When the microneedle sheet 1 is used, the substrate layer of the microneedle sheet 1 is attached to the application surface of the curved side surface of the roller 2-1, as shown in Figure 3, and the applicator 2 with the microneedle sheet 1 is applied onto a keratin substance 3, such as the skin, such that the roller 2-1 can rotate on the keratin substance 3.

The microneedles of the microneedle sheet 1 on the applicator 2 can penetrate into the keratin substance to provide the keratin substance with cosmetic effects derived from the microneedles.

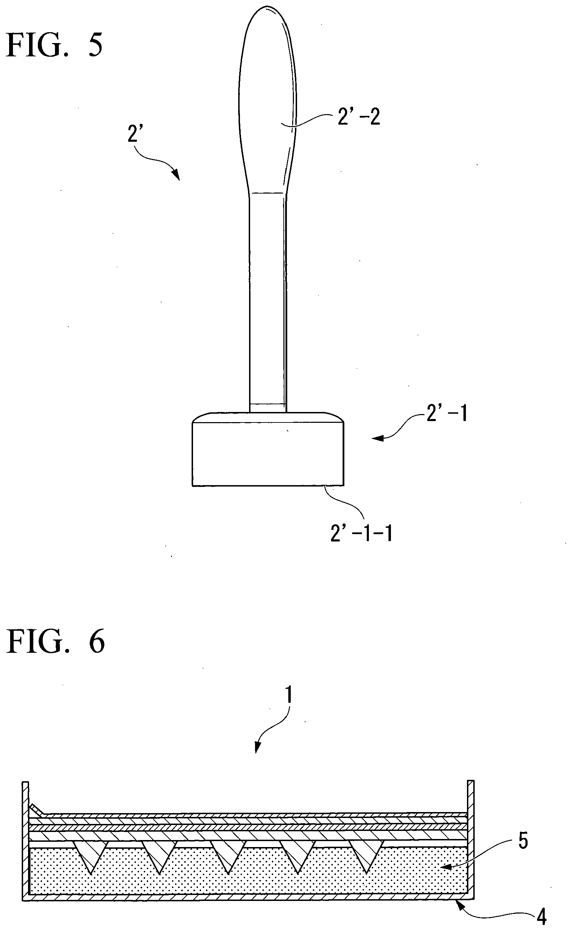

Figure 5 shows an example of an applicator having a flat application surface (not claimed) The applicator 2' shown in Figure 5 has a stamp 2'-1 having a flat bottom surface 2'-1-1, and the flat bottom surface 2'-1-1 of the stamp 2'-1 comprises an application surface to which the substrate layer of a microneedle sheet can be attached.

The applicator 2' also has a handle 2'-2. A user of the applicator 2' can grasp the handle 2'-2 to use the applicator 2'.

When a microneedle sheet is used, the microneedle sheet is attached to the application surface on the flat bottom surface 2'-1-1 of the stamp 2'-1 such that the substrate layer of the microneedle sheet contacts the application surface. Thus, the microneedles of the microneedle sheet can stand up on the flat bottom surface 2'-1-1of the stamp 2'-1.

At least a part of the flat bottom surface 2'-1-1of the stamp 2'-1 may be covered by the microneedle sheet. It is preferable that the entirety of the flat bottom surface 2'-1-1of the stamp 2'-1 be covered by the microneedle sheet.

After the substrate layer of the microneedle sheet is attached to the application surface of the flat bottom surface 2'-1-1 of the stamp 2'-1, the applicator 2' with the microneedle sheet is applied onto the keratin substance 3, such as the skin, such that the flat bottom surface 2'-1-1 of the stamp 2'-1 with the microneedles can be stamped onto a keratin substance such as skin.

The microneedles of the microneedle sheet on the applicator 2' can penetrate into the keratin substance to provide the keratin substance with cosmetic effects derived from the microneedles.

### { Container}

The kit according to the present invention may comprise at least one container.

The container can include the microneedle sheet in order to protect the microneedle sheet, in particular the microneedles of the microneedle sheet. Therefore, it is possible to perform safe storage of the microneedle sheet without breaking the microneedles.

It is preferable that the container comprise at least one soft and/or porous article. The microneedles of the microneedle sheet can safely penetrate into the soft article or can be placed in the pores of the porous article when not being used.

The soft and/or porous article can reduce or eliminate damage to the microneedles. Accordingly, the use of the soft and/or porous article can protect the microneedles in a safer manner.

The soft article may be composed of at least one soft synthetic resin. The soft synthetic resin may be selected from polyurethanes, polystyrenes, polycarbonates, polyethylene terephthalates, poly(ethylene-co-vinyl alcohol), polyethylenes, polyesters, polyamides, and combinations thereof, which may include at least one softening agent.

The porous article may be composed of fibers. The fibers may be made from natural fibers such as cotton, semi-natural fibers such as viscose, synthetic fibers such as polyester and polypropylene fibers, and combinations thereof. The porous article may be selected from woven or non-woven fabrics.

The soft and porous article may be composed of sponges. The sponges may be made of foamed synthetic resins such as foamed flexible polyurethanes.

It is preferable that the soft and/or porous article be in the form of a sheet. The soft and/or porous sheet should have a thickness which is greater than the height of the microneedles. For example, if the microneedles have a height of 100 to 400 microns, the thickness of the soft or porous sheet should be 200 to 500 microns.

Figure 6 shows an example of a container. In Figure 6, a container 4 is in the shape of a tray with an opening. The material of the container 4 is not limited. For example, at least one selected from metals and synthetic resins may be used as the material of the container 4.

In the example shown in Figure 6, the container 4 includes a porous sheet 5. The porous sheet 5 is made from a sponge or a woven or non-woven fabric.

As shown in Figure 6, when the microneedle sheet 1 is not used, the microneedle sheet 1 is stored in the container 4, and the microneedles of the microneedle sheet 1 may penetrate into the porous sheet 5 to be protected. Thus, the microneedle sheet 1 can be stored with the protection of microneedles.

It may be preferable that the microneedle sheet in the container be packaged by covering the opening of the container 4 or both the opening of the container 4 and the container 4 itself with, for example, a thin film such as a thin transparent resin film.

### {Cosmetic Composition}

The kit according to the present invention may comprise at least one cosmetic composition.

The cosmetic composition may be used as a pre-treatment or post-treatment composition for a keratin substance.

The cosmetic composition may comprises at least one oil and water, preferably in the form of an O/W emulsion.

### (Oil)

The cosmetic composition which may be used for the present invention may comprise at least one oil. If two or more oils are used, they may be the same or different.

Here, "oil" means a fatty compound or substance which is in the form of a liquid or a paste (non-solid) at room temperature (25°C) under atmospheric pressure (760 mmHg). As the oils, those generally used in cosmetics can be used alone or in combination thereof. These oils may be volatile or non-volatile.

The oil may be a non-polar oil such as a hydrocarbon oil, a silicone oil, or the like; a polar oil such as a plant or animal oil and an ester oil or an ether oil; or a mixture thereof.

The oil may be selected from the group consisting of oils of plant or animal origin, synthetic oils, silicone oils, hydrocarbon oils, and fatty alcohols.

As examples of plant oils, mention may be made of, for example, linseed oil, camellia oil, macadamia nut oil, corn oil, mink oil, olive oil, avocado oil, sasanqua oil, castor oil, safflower oil, jojoba oil, sunflower oil, almond oil, rapeseed oil, sesame oil, soybean oil, peanut oil, and mixtures thereof.

As examples of animal oils, mention may be made of, for example, squalene and squalane.

As examples of synthetic oils, mention may be made of alkane oils such as isododecane and isohexadecane, ester oils, ether oils, and artificial triglycerides.

The ester oils are preferably liquid esters of saturated or unsaturated, linear or branched C₁-C₂₆ aliphatic monoacids or polyacids and of saturated or unsaturated, linear or branched C₁-C₂₆ aliphatic monoalcohols or polyalcohols, the total number of carbon atoms of the esters being greater than or equal to 10.

Preferably, for the esters of monoalcohols, at least one from among the alcohol and the acid from which the esters of the present invention are derived is branched.

Among the monoesters of monoacids and of monoalcohols, mention may be made of ethyl palmitate, ethyl hexyl palmitate, isopropyl palmitate, dicaprylyl carbonate, alkyl myristates such as isopropyl myristate or ethyl myristate, isocetyl stearate, 2-ethylhexyl isononanoate, isononyl isononanoate, isodecyl neopentanoate, and isostearyl neopentanoate.

Esters of C₄-C₂₂ dicarboxylic or tricarboxylic acids and of C₁-C₂₂ alcohols, and esters of monocarboxylic, dicarboxylic, or tricarboxylic acids and of non-sugar C₄-C₂₆ dihydroxy, trihydroxy, tetrahydroxy, or pentahydroxy alcohols may also be used.

Mention may especially be made of: diethyl sebacate; isopropyl lauroyl sarcosinate; diisopropyl sebacate; bis(2-ethylhexyl) sebacate; diisopropyl adipate; di-n-propyl adipate; dioctyl adipate; bis(2-ethylhexyl) adipate; diisostearyl adipate; bis(2-ethylhexyl) maleate; triisopropyl citrate; triisocetyl citrate; triisostearyl citrate; glyceryl trilactate; glyceryl trioctanoate; trioctyldodecyl citrate; trioleyl citrate; neopentyl glycol diheptanoate; diethylene glycol diisononanoate.

As ester oils, one can use sugar esters and diesters of C₆-C₃₀ and preferably C₁₂-C₂₂ fatty acids. It is recalled that the term "sugar" means oxygen-bearing hydrocarbon-based compounds containing several alcohol functions, with or without aldehyde or ketone functions, and which comprise at least 4 carbon atoms. These sugars may be monosaccharides, oligosaccharides, or polysaccharides.

Examples of suitable sugars that may be mentioned include sucrose (or saccharose), glucose, galactose, ribose, fucose, maltose, fructose, mannose, arabinose, xylose, and lactose, and derivatives thereof, especially alkyl derivatives, such as methyl derivatives, for instance methylglucose.

The sugar esters of fatty acids may be chosen especially from the group comprising the esters or mixtures of esters of sugars described previously and of linear or branched, saturated or unsaturated C₆-C₃₀ and preferably C₁₂-C₂₂ fatty acids. If they are unsaturated, these compounds may have one to three conjugated or non-conjugated carbon-carbon double bonds.

The esters according to this variant may also be selected from monoesters, diesters, triesters, tetraesters, and polyesters, and mixtures thereof.

These esters may be, for example, oleates, laurates, palmitates, myristates, behenates, cocoates, stearates, linoleates, linolenates, caprates, and arachidonates, or mixtures thereof such as, especially, oleopalmitate, oleostearate, and palmitostearate mixed esters, as well as pentaerythrityl tetraethyl hexanoate.

More particularly, use is made of monoesters and diesters and especially sucrose, glucose, or methylglucose monooleates or dioleates, stearates, behenates, oleopalmitates, linoleates, linolenates, and oleostearates.

An example that may be mentioned is the product sold under the name Glucate^{®} DO by the company Amerchol, which is a methylglucose dioleate.

As examples of preferable ester oils, mention may be made of, for example, diisopropyl adipate, dioctyl adipate, 2-ethylhexyl hexanoate, ethyl laurate, cetyl octanoate, octyldodecyl octanoate, isodecyl neopentanoate, myristyl propionate, 2-ethylhexyl 2-ethylhexanoate, 2-ethylhexyl octanoate, 2-ethylhexyl caprylate/caprate, methyl palmitate, ethyl palmitate, isopropyl palmitate, dicaprylyl carbonate, isopropyl lauroyl sarcosinate, isononyl isononanoate, ethylhexyl palmitate, isohexyl laurate, hexyl laurate, isocetyl stearate, isopropyl isostearate, isopropyl myristate, isodecyl oleate, glyceryl tri(2-ethylhexanoate), pentaerythrithyl tetra(2-ethylhexanoate), 2-ethylhexyl succinate, diethyl sebacate, and mixtures thereof.

As examples of artificial triglycerides, mention may be made of, for example, capryl caprylyl glycerides, glyceryl trimyristate, glyceryl tripalmitate, glyceryl trilinolenate, glyceryl trilaurate, glyceryl tricaprate, glyceryl tricaprylate, glyceryl tri(caprate/caprylate), and glyceryl tri(caprate/caprylate/linolenate).

As examples of silicone oils, mention may be made of, for example, linear organopolysiloxanes such as dimethylpolysiloxane, methylphenylpolysiloxane, methylhydrogenpolysiloxane, and the like; cyclic organopolysiloxanes such as cyclohexasiloxane, octamethylcyclotetrasiloxane, decamethylcyclopentasiloxane, dodecamethylcyclohexasiloxane, and the like; and mixtures thereof.

Preferably, the silicone oil is chosen from liquid polydialkylsiloxanes, especially liquid polydimethylsiloxanes (PDMS) and liquid polyorganosiloxanes comprising at least one aryl group.

These silicone oils may also be organomodified. The organomodified silicones that can be used in accordance with the present invention are silicone oils as defined above and comprise in their structure one or more organofunctional groups attached via a hydrocarbon-based group.

Organopolysiloxanes are defined in greater detail in Walter Noll's Chemistry and Technology of Silicones (1968), Academic Press. They may be volatile or non-volatile.

When they are volatile, the silicones are more particularly chosen from those having a boiling point of between 60°C and 260°C, and even more particularly from:
(i) cyclic polydialkylsiloxanes comprising from 3 to 7 and preferably 4 to 5 silicon atoms. These are, for example, octamethylcyclotetrasiloxane sold in particular under the name Volatile Silicone^{®} 7207 by Union Carbide or Silbione^{®} 70045 V2 by Rhodia, decamethylcyclopentasiloxane sold under the name Volatile Silicone^{®} 7158 by Union Carbide, Silbione^{®} 70045 V5 by Rhodia, and dodecamethylcyclopentasiloxane sold under the name Silsoft 1217 by Momentive Performance Materials, and mixtures thereof. Mention may also be made of cyclocopolymers of the type such as dimethylsiloxane/methylalkylsiloxane, such as Silicone Volatile^{®} FZ 3109 sold by the company Union Carbide, of the formula: 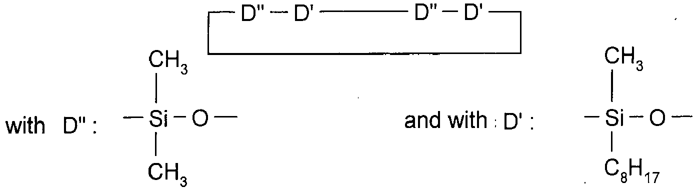 Mention may also be made of mixtures of cyclic polydialkylsiloxanes with organosilicon compounds, such as the mixture of octamethylcyclotetrasiloxane and tetratrimethylsilylpentaerythritol (50/50) and the mixture of octamethylcyclotetrasiloxane and oxy-1,1'-bis(2,2,2',2',3,3'-hexatrimethylsilyloxy)neopentane; and
(ii) linear volatile polydialkylsiloxanes containing 2 to 9 silicon atoms and having a viscosity of less than or equal to 5×10⁻⁶ m²/s at 25°C. An example is decamethyltetrasiloxane sold in particular under the name SH 200 by the company Toray Silicone. Silicones belonging to this category are also described in the article published in Cosmetics and Toiletries, Vol. 91, Jan. 76, pp. 27-32, Todd & Byers, Volatile Silicone Fluids for Cosmetics*.* The viscosity of the silicones is measured at 25°C according to ASTM standard 445 Appendix C.

Non-volatile polydialkylsiloxanes may also be used. These non-volatile silicones are more particularly chosen from polydialkylsiloxanes, among which mention may be made mainly of polydimethylsiloxanes containing trimethylsilyl end groups.

Among these polydialkylsiloxanes, mention may be made, in a non-limiting manner, of the following commercial products:
- the Silbione^{®} oils of the 47 and 70 047 series or the Mirasil^{®} oils sold by Rhodia, for instance the oil 70 047 V 500 000;
- the oils of the Mirasil^{®} series sold by the company Rhodia;
- the oils of the 200 series from the company Dow Coming, such as DC200 with a viscosity of 60 000 mm²/s; and
- the Viscasil^{®} oils from General Electric and certain oils of the SF series (SF 96, SF 18) from General Electric.

Mention may also be made of polydimethylsiloxanes containing dimethylsilanol end groups known under the name dimethiconol (CTFA), such as the oils of the 48 series from the company Rhodia.

Among the silicones containing aryl groups, mention may be made of polydiarylsiloxanes, especially polydiphenylsiloxanes and polyalkylarylsiloxanes such as phenyl silicone oil.

The phenyl silicone oil may be chosen from the phenyl silicones of the following formula: 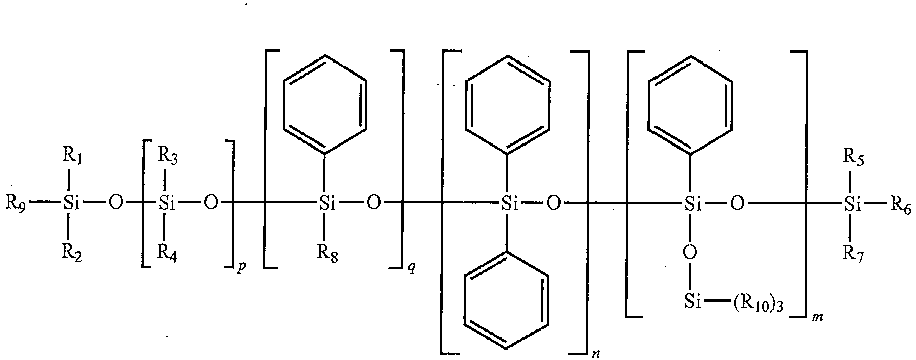 in which
R₁ to R₁₀, independently of each other, are saturated or unsaturated, linear, cyclic or branched C₁-C₃₀ hydrocarbon-based radicals, preferably C₁-C₁₂ hydrocarbon-based radicals, and more preferably C₁-C₆ hydrocarbon-based radicals, in particular methyl, ethyl, propyl, or butyl radicals, and
m, n, p, and q are, independently of each other, integers of 0 to 900 inclusive, preferably 0 to 500 inclusive, and more preferably 0 to 100 inclusive,
with the proviso that the sum n+m+q is other than 0.

Examples that may be mentioned include the products sold under the following names:
- the Silbione^{®} oils of the 70 641 series from Rhodia;
- the oils of the Rhodorsil^{®} 70 633 and 763 series from Rhodia;
- the oil Dow Corning 556 Cosmetic Grade Fluid from Dow Coming;
- the silicones of the PK series from Bayer, such as the product PK20;
- certain oils of the SF series from General Electric, such as SF 1023, SF 1154, SF 1250, and SF 1265.

As the phenyl silicone oil, phenyl trimethicone (R₁ to R₁₀ are methyl; p, q, and n = 0; m=1 in the above formula) is preferable.

The organomodified liquid silicones may especially contain polyethyleneoxy and/or polypropyleneoxy groups. Mention may thus be made of the silicone KF-6017 proposed by Shin-Etsu, and the oils Silwet^{®} L722 and L77 from the company Union Carbide.

Hydrocarbon oils may be chosen from:
- linear or branched, optionally cyclic, C₆-C₁₆ lower alkanes. Examples that may be mentioned include hexane, undecane, dodecane, tridecane, and isoparaffins, for instance isohexadecane, isododecane, and isodecane; and
- linear or branched hydrocarbons containing more than 16 carbon atoms, such as liquid paraffins, liquid petroleum jelly, polydecenes and hydrogenated polyisobutenes such as Parleam^{®}, and squalane.

As preferable examples of hydrocarbon oils, mention may be made of, for example, linear or branched hydrocarbons such as isohexadecane, isododecane, squalane, mineral oil (e.g., liquid paraffin), paraffin, vaseline or petrolatum, naphthalenes, and the like; hydrogenated polyisobutene, isoeicosan, and decene/butene copolymer; and mixtures thereof.

The term "fatty" in the fatty alcohol means the inclusion of a relatively large number of carbon atoms. Thus, alcohols which have 4 or more, preferably 6 or more, and more preferably 12 or more carbon atoms are encompassed within the scope of fatty alcohols. The fatty alcohol may be saturated or unsaturated. The fatty alcohol may be linear or branched.

The fatty alcohol may have the structure R-OH wherein R is chosen from saturated and unsaturated, linear and branched radicals containing from 4 to 40 carbon atoms, preferably from 6 to 30 carbon atoms, and more preferably from 12 to 20 carbon atoms. In at least one embodiment, R may be chosen from C₁₂-C₂₀ alkyl and C₁₂-C₂₀ alkenyl groups. R may or may not be substituted with at least one hydroxyl group.

As examples of the fatty alcohol, mention may be made of lauryl alcohol, cetyl alcohol, stearyl alcohol, isostearyl alcohol, behenyl alcohol, undecylenyl alcohol, myristyl alcohol, octyldodecanol, hexyldecanol, oleyl alcohol, linoleyl alcohol, palmitoleyl alcohol, arachidonyl alcohol, erucyl alcohol, and mixtures thereof.

It is preferable that the fatty alcohol be a saturated fatty alcohol.

Thus, the fatty alcohol may be selected from straight or branched, saturated or unsaturated C₆-C₃₀ alcohols, preferably straight or branched, saturated C₆-C₃₀ alcohols, and more preferably straight or branched, saturated C₁₂-C₂₀ alcohols.

The term "saturated fatty alcohol" here means an alcohol having a long aliphatic saturated carbon chain. It is preferable that the saturated fatty alcohol be selected from any linear or branched, saturated C₆-C₃₀ fatty alcohols. Among the linear or branched, saturated C₆-C₃₀ fatty alcohols, linear or branched, saturated C₁₂-C₂₀ fatty alcohols may preferably be used. Any linear or branched, saturated C₁₆-C₂₀ fatty alcohols may be more preferably used. Branched C₁₆-C₂₀ fatty alcohols may be even more preferably used.

As examples of saturated fatty alcohols, mention may be made of lauryl alcohol, cetyl alcohol, stearyl alcohol, isostearyl alcohol, behenyl alcohol, undecylenyl alcohol, myristyl alcohol, octyldodecanol, hexyldecanol, and mixtures thereof. In one embodiment, cetyl alcohol, stearyl alcohol, octyldodecanol, hexyldecanol, or a mixture thereof (e.g., cetearyl alcohol) as well as behenyl alcohol, can be used as a saturated fatty alcohol.

According to at least one embodiment, the fatty alcohol used in the cosmetic composition which may be used for the present invention is preferably chosen from cetyl alcohol, octyldodecanol, hexyldecanol, and mixtures thereof.

It may be preferable that the oil be chosen from ester oils, hydrocarbon oils, silicone oils, fatty alcohols, and mixtures thereof.

The amount of the oil(s) in the cosmetic composition which may be used for the present invention may be 1% by weight or more, preferably 5% by weight or more, and more preferably 10% by weight or more, relative to the total weight of the cosmetic composition. The amount of the oil(s) in the cosmetic composition which may be used for the present invention may be 30% by weight or less, preferably 25% by weight or less, and more preferably 20% by weight or less, relative to the total weight of the cosmetic composition. The amount of the oil(s) in the cosmetic composition which may be used for the present invention may be from 1% to 30% by weight, preferably from 5% to 25% by weight, and more preferably from 10% to 20% by weight, relative to the total weight of the cosmetic composition.

The oil(s) can form a fatty phase of the cosmetic composition which may be used for the present invention.

If the cosmetic composition which may be used for the present invention is in the form of an O/W emulsion, the oil(s) can form dispersed fatty phases in the O/W emulsion.

### (Water)

The cosmetic composition which may be used for the present invention may comprise water.

The amount of the water may be 50% by weight or more, preferably 55% by weight or more, and more preferably 60% by weight or more, relative to the total weight of the cosmetic composition. The amount of the water may be 95% by weight or less, preferably 90% by weight or less, and more preferably 85% by weight or less, relative to the total weight of the cosmetic composition. The amount of water in the cosmetic composition which may be used for the present invention may range from 50% to 95% by weight, preferably from 55% to 90% by weight, and more preferably from 60% to 85% by weight, relative to the total weight of the cosmetic composition.

The water can form an aqueous phase of the cosmetic composition which may be used for the present invention.

If the cosmetic composition which may be used for the present invention is in the form of an O/W emulsion, the water can form continuous aqueous phases in the O/W emulsion.

### (Surfactant)

The cosmetic composition which may be used for the present invention may include at least one surfactant. If two or more surfactants are used, they may be the same or different.

Any surfactant may be used for the present invention. The surfactant which may be used in the present invention may be selected from the group consisting of anionic surfactants, amphoteric surfactants, cationic surfactants, and nonionic surfactants. Two or more surfactants may be used in combination. Thus, a single type of surfactant or a combination of different types of surfactants may be used.

### Anionic Surfactants

According to the present invention, the type of anionic surfactant is not limited. It is preferable that the anionic surfactant be selected from the group consisting of (C₆-C₃₀)alkyl sulfates, (C₆-C₃₀)alkyl ether sulfates, (C₆-C₃₀)alkylamido ether sulfates, alkylaryl polyether sulfates, and monoglyceride sulfates; (C₆-C₃₀)alkylsulfonates, (C₆-C₃₀)alkylamide sulfonates, (C₆-C₃₀)alkylaryl sulfonates, α-olefin sulfonates, and paraffin sulfonates; (C₆-C₃₀)alkyl phosphates; (C₆-C₃₀)alkyl sulfosuccinates, (C₆-C₃₀)alkyl ether sulfosuccinates, and (C₆-C₃₀)alkylamide sulfosuccinates; (C₆-C₃₀)alkyl sulfoacetates; (C₆-C₂₄)acyl sarcosinates; (C₆-C₂₄)acyl glutamates; (C₆-C₃₀)alkylpolyglycoside carboxylic ethers; (C₆-C₃₀)alkylpolyglycoside sulfosuccinates; (C₆-C₃₀)alkyl sulfosuccinamates; (C₆-C₂₄)acyl isethionates; N-(C₆-C₂₄)acyl taurates; C₆-C₃₀ fatty acid salts; coconut oil acid salts or hydrogenated coconut oil acid salts; (C₈-C₂₀)acyl lactylates; (C₆-C₃₀)alkyl-D-galactoside uronic acid salts; polyoxyalkylenated (C₆-C₃₀)alkyl ether carboxylic acid salts; polyoxyalkylenated (C₆-C₃₀)alkylaryl ether carboxylic acid salts; and polyoxyalkylenated (C₆-C₃₀)alkylamido ether carboxylic acid salts.

It is more preferable that the anionic surfactant be selected from salts of (C₆-C₃₀)alkyl sulfates or polyoxyalkylenated (C₆-C₃₀)alkyl ether carboxylic acid salts.

In at least one embodiment, the anionic surfactants are in the form of salts such as salts of alkali metals, for instance sodium; salts of alkaline-earth metals, for instance magnesium; ammonium salts; amine salts; and amino alcohol salts. Depending on the conditions, they may also be in acid form.

### Amphoteric Surfactants

According to the present invention, the type of amphoteric surfactant is not limited. The amphoteric or zwitterionic surfactants can be, for example (non-limiting list), amine derivatives such as aliphatic secondary or tertiary amine, and optionally quaternized amine derivatives, in which the aliphatic radical is a linear or branched chain comprising 8 to 22 carbon atoms and containing at least one water-solubilizing anionic group (for example, carboxylate, sulphonate, sulphate, phosphate, or phosphonate).

The amphoteric surfactant may preferably be selected from the group consisting of betaines and amidoaminecarboxylated derivatives.

The betaine-type amphoteric surfactant is preferably selected from the group consisting of alkylbetaines, alkylamidoalkylbetaines, sulfobetaines, phosphobetaines, and alkylamidoalkylsulfobetaines, in particular, (C₈-C₂₄)alkylbetaines, (C₈-C₂₄)alkylamido(C₁-Cs)alkylbetaines, sulphobetaines, and (C₈-C₂₄)alkylamido(C₁-C₈)alkylsulphobetaines. In one embodiment, the amphoteric surfactants of betaine type are chosen from (C₈-C₂₄)alkylbetaines, (C₈-C₂₄)alkylamido(C₁-C₈)alkylsulphobetaines, sulphobetaines, and phosphobetaines.

Non-limiting examples that may be mentioned include the compounds classified in the CTFA dictionary, 9th edition, 2002, under the names cocobetaine, laurylbetaine, cetylbetaine, coco/oleamidopropylbetaine, cocamidopropylbetaine, palmitamidopropylbetaine, stearamidopropylbetaine, cocamidoethylbetaine, cocamidopropylhydroxysultaine, oleamidopropylhydroxysultaine, cocohydroxysultaine, laurylhydroxysultaine, and cocosultaine, alone or as mixtures.

The betaine-type amphoteric surfactant is preferably an alkylbetaine and an alkylamidoalkylbetaine, in particular cocobetaine and cocamidopropylbetaine.

Among the amidoaminecarboxylated derivatives, mention may be made of the products sold under the name Miranol, as described in U.S. Pat. Nos. 2,528,378 and 2,781,354 and classified in the CTFA dictionary, 3rd edition, 1982 (the disclosures of which are incorporated herein by reference), under the names Amphocarboxyglycinates and Amphocarboxypropionates, with the respective structures:

R₁-CONHCH₂CH₂-N⁺(R₂)(R₃)(CH₂COO⁻)

in which:
R₁ denotes an alkyl radical of an acid R₁-COOH present in hydrolysed coconut oil, a heptyl, nonyl, or undecyl radical,
R₂ denotes a beta-hydroxyethyl group, and
R₃ denotes a carboxymethyl group; and

   R₁'-CONHCH₂CH₂-N(B)(C)

   in which:
   B represents -CH₂CH₂OX',
   C represents -(CH₂)_{z}-Y', with z=1 or 2,
   X' denotes a -CH₂CH₂-COOH group, -CH₂-COOZ', -CH₂CH₂-COOH, -CH₂CH₂-COOZ', or a hydrogen atom,
   Y' denotes -COOH, -COOZ', -CH₂-CHOH-SO₃Z', or a -CH₂-CHOH-SO₃H radical,
   Z' represents an ion of an alkaline or alkaline earth metal such as sodium, an ammonium ion, or an ion issued from an organic amine, and
   R₁' denotes an alkyl radical of an acid R₁'-COOH present in coconut oil or in hydrolysed linseed oil, an alkyl radical, such as a C₇, C₉, C₁₁, or C₁₃ alkyl radical, a C₁₇ alkyl radical and its iso form, or an unsaturated C₁₇ radical.

It is preferable that the amphoteric surfactant be selected from (C₈-C₂₄)-alkyl amphomonoacetates, (C₈-C₂₄)alkyl amphodiacetates, (C₈-C₂₄)alkyl amphomonopropionates, and (C₈-C₂₄)alkyl amphodipropionates

These compounds are classified in the CTFA dictionary, 5th edition, 1993, under the names Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphopropionate, Disodium Caprylamphodipropionate, Disodium Caprylamphodipropionate, Lauroamphodipropionic acid, and Cocoamphodipropionic acid.

By way of example, mention may be made of the cocoamphodiacetate sold under the trade name Miranol^{®} C2M concentrate by the company Rhodia Chimie.

### Cationic Surfactants

According to the present invention, the type of cationic surfactant is not limited. The cationic surfactant may be selected from the group consisting of optionally polyoxyalkylenated, primary, secondary, or tertiary fatty amine salts, quaternary ammonium salts, and mixtures thereof.

Examples of quaternary ammonium salts that may be mentioned include, but are not limited to:
those of general formula (I) below: 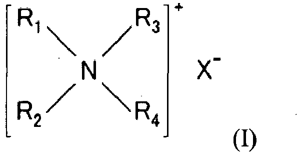 wherein
R₁, R₂, R₃, and R₄, which may be identical or different, are chosen from linear and branched aliphatic radicals comprising from 1 to 30 carbon atoms and optionally comprising heteroatoms such as oxygen, nitrogen, sulfur, and halogens. The aliphatic radicals may be chosen, for example, from alkyl, alkoxy, C₂-C₆ polyoxyalkylene, alkylamide, (C₁₂-C₂₂)alkylamido(C₂-C₆)alkyl, (C₁₂-C₂₂)alkylacetate, and hydroxyalkyl radicals; and aromatic radicals such as aryl and alkylaryl; and X⁻ is chosen from halides, phosphates, acetates, lactates, (C₂-C₆) alkyl sulfates, and alkyl- or alkylaryl-sulfonates;
quaternary ammonium salts of imidazoline, for instance those of formula (II) below: 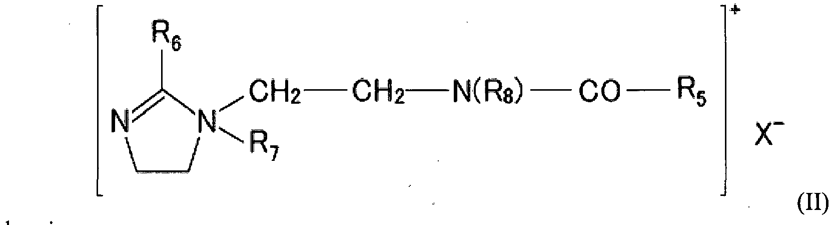 wnerem:
   R₅ is chosen from alkenyl and alkyl radicals comprising from 8 to 30 carbon atoms, for example fatty acid derivatives of tallow or of coconut;
   R₆ is chosen from hydrogen, C₁-C₄ alkyl radicals, and alkenyl and alkyl radicals comprising from 8 to 30 carbon atoms;
   R₇ is chosen from C₁-C₄ alkyl radicals;
   R₈ is chosen from hydrogen and C₁-C₄ alkyl radicals; and
   X⁻ is chosen from halides, phosphates, acetates, lactates, alkyl sulfates, alkyl sulfonates, and
   alkylaryl sulfonates. In one embodiment, R₅ and R₆ are, for example, a mixture of radicals chosen from alkenyl and alkyl radicals comprising from 12 to 21 carbon atoms, such as fatty acid derivatives of tallow, R₇ is methyl, and R₈ is hydrogen. Examples of such products include, but are not limited to, Quaternium-27 (CTFA 1997) and Quaternium-83 (CTFA 1997),
   which are sold under the names "Rewoquat^{®}" W75, W90, W75PG, and W75HPG by the company Witco;
   diquaternary ammonium salts of formula (III): 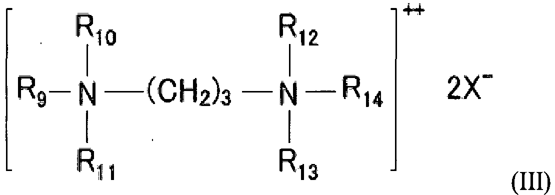 wherein:
      R₉ is chosen from aliphatic radicals comprising from 16 to 30 carbon atoms;
      R₁₀ is chosen from hydrogen or alkyl radicals comprising from 1 to 4 carbon atoms or the group (R₁₆ₐ)(R₁₇ₐ)(R₁₈ₐ)N⁺(CH₂)₃;
      R₁₁, R₁₂, R₁₃, R₁₄, R₁₆ₐ, R₁₇ₐ, and R₁₈ₐ, which may be identical or different, are chosen from hydrogen and alkyl radicals comprising from 1 to 4 carbon atoms; and
      X⁻ is chosen from halides, acetates, phosphates, nitrates, ethyl sulfates, and methyl sulfates. An example of one such diquaternary ammonium salt is FINQUAT CT-P of FINETEX (Quaternium-89) or FINQUAT CT of FINETEX (Quaternium-75); and
      quaternary ammonium salts comprising at least one ester function, such as those of formula (IV) below: 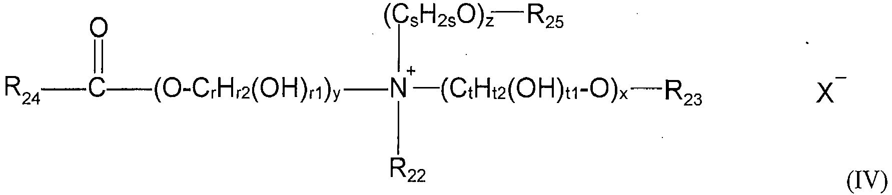 wherein:
         R₂₂ is chosen from Ci-C6 alkyl radicals, and C₁-C₆ hydroxyalkyl and dihydroxyalkyl radicals;
         R₂₃ is chosen from:
            the radical below: 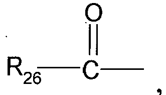
            linear and branched, saturated and unsaturated C₁-C₂₂ hydrocarbon-based radicals R₂₇, and hydrogen,
            R₂₅ is chosen from:
            the radical below: 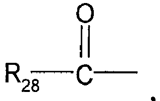
            linear and branched, saturated and unsaturated C₁-C₆ hydrocarbon-based radicals R₂₉, and hydrogen,
            R₂₄, R₂₆, and R₂₈, which may be identical or different, are chosen from linear and branched, saturated and unsaturated, C₇-C₂₁, hydrocarbon-based radicals;
            r, s, and t, which may be identical or different, are chosen from integers ranging from 2 to 6; each of r1 and t1, which may be identical or different, is 0 or 1, and r2+r1=2r and t1+2t=2t; y is chosen from integers ranging from 1 to 10;
            x and z, which may be identical or different, are chosen from integers ranging from 0 to 10;
            X⁻ is chosen from simple and complex, organic and inorganic anions; with the proviso that the sum x+y+z ranges from 1 to 15, that when x is 0, R₂₃ denotes R₂₇, and that when z is 0, R₂₅ denotes R₂₉. R₂₂ may be chosen from linear and branched alkyl radicals. In one embodiment, R₂₂ is chosen from linear alkyl radicals. In another embodiment, R₂₂ is chosen from methyl, ethyl, hydroxyethyl, and dihydroxypropyl radicals, for example methyl and ethyl radicals. In one embodiment, the sum x+y+z ranges from 1 to 10. When R₂₃ is a hydrocarbon-based radical R₂₇, it may be long and comprise from 12 to 22 carbon atoms, or short and comprise from 1 to 3 carbon atoms. When R₂₅ is a hydrocarbon-based radical R₂₉, it may comprise, for example, from 1 to 3 carbon atoms. By way of a non-limiting example, in one embodiment, R₂₄, R₂₆, and R₂₈, which may be identical or different, are chosen from linear and branched, saturated and unsaturated, C₁₁-C₂₁ hydrocarbon-based radicals, for example from linear and branched, saturated and unsaturated C₁₁-C₂₁ alkyl and alkenyl radicals. In another embodiment, x and z, which may be identical or different, are 0 or 1. In one embodiment, y is equal to 1. In another embodiment, r, s, and t, which may be identical or different, are equal to 2 or 3, for example equal to 2. The anion X⁻ may be chosen from, for example, halides, such as chloride, bromide, and iodide; and C₁-C₄ alkyl sulfates, such as methyl sulfate. However, methanesulfonate, phosphate, nitrate, tosylate, an anion derived from an organic acid, such as acetate and lactate, and any other anion that is compatible with the ammonium comprising an ester function, are other non-limiting examples of anions that may be used according to the present invention. In one embodiment, the anion X⁻ is chosen from chloride and methyl sulfate.

In another embodiment, the ammonium salts of formula (IV) may be used, wherein:
R₂₂ is chosen from methyl and ethyl radicals,
x and y are equal to 1;
z is equal to 0 or 1;
r, s, and t are equal to 2;
R₂₃ is chosen from:
the radical below: 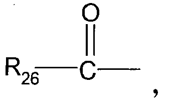 J methyl, ethyl, and C₁₄-C₂₂ hydrocarbon-based radicals, and hydrogen; R₂₅ is chosen from:
the radical below: 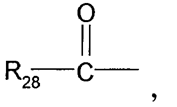 and hydrogen;
R₂₄, R₂₆, and R₂₈, which may be identical or different, are chosen from linear and branched, saturated and unsaturated, C₁₃-C₁₇ hydrocarbon-based radicals, for example from linear and branched, saturated and unsaturated, C₁₃-C₁₇ alkyl and alkenyl radicals.

In one embodiment, the hydrocarbon-based radicals are linear.

Non-limiting examples of compounds of formula (IV) that may be mentioned include salts, for example chloride and methyl sulfate, of diacyloxyethyl-dimethylammonium, of diacyloxyethyl-hydroxyethyl-methylammonium, of monoacyloxyethyl-dihydroxyethyl-methylammonium, of triacyloxyethyl-methylammonium, of monoacyloxyethyl-hydroxyethyl-dimethyl-ammonium, and mixtures thereof. In one embodiment, the acyl radicals may comprise from 14 to 18 carbon atoms, and may be derived, for example, from a plant oil, for instance palm oil and sunflower oil. When the compound comprises several acyl radicals, these radicals may be identical or different.

These products may be obtained, for example, by direct esterification of optionally oxyalkylenated triethanolamine, triisopropanolamine, alkyldiethanolamine, or alkyldiisopropanolamine onto fatty acids or onto mixtures of fatty acids of plant or animal origin, or by transesterification of the methyl esters thereof. This esterification may be followed by a quaternization using an alkylating agent chosen from alkyl halides, for example methyl and ethyl halides; dialkyl sulfates, for example dimethyl and diethyl sulfates; methyl methanesulfonate; methyl para-toluenesulfonate; glycol chlorohydrin; and glycerol chlorohydrin.

Such compounds are sold, for example, under the names Dehyquart^{®} by the company Cognis, Stepanquat^{®} by the company Stepan, Noxamium^{®} by the company Ceca, and "Rewoquat^{®} WE 18" by the company Rewo-Goldschmidt.

Other non-limiting examples of ammonium salts that may be used in the cosmetic composition which may be used for the present invention include the ammonium salts comprising at least one ester function described in U.S. Pat. Nos. 4,874,554 and 4,137,180.

The quaternary ammonium salts mentioned above that may be used in the cosmetic composition which may be used for the present invention include, but are not limited to, those corresponding to formula (I), for example tetraalkylammonium chlorides, for instance dialkyldimethylammonium and alkyltrimethylammonium chlorides in which the alkyl radical comprises from about 12 to 22 carbon atoms, such as behenyltrimethylammonium, distearyldimethylammonium, cetyltrimethylammonium, and benzyldimethylstearylammonium chloride; palmitylamidopropyltrimethylammonium chloride; and stearamidopropyldimethyl(myristyl acetate)ammonium chloride, sold under the name "Ceraphyl^{®} 70" by the company Van Dyk.

According to one embodiment, the cationic surfactant that may be used in the cosmetic composition which may be used for the present invention is chosen from quaternary ammonium salts, for example from behenyltrimethylammonium chloride, cetyltrimethylammonium chloride, Quaternium-83, Quaternium-87, Quaternium-22, behenylamidopropyl-2,3-dihydroxypropyldimethylammonium chloride, palmitylamidopropyltrimethylammonium chloride, and stearamidopropyldimethylamine.

### Nonionic Surfactants

Nonionic surfactants are compounds well known in and of themselves (see, e.g., in this regard, "Handbook of Surfactants" by M. R. Porter, Blackie & Son publishers (Glasgow and London), 1991, pp. 116-178). Thus, they can, for example, be chosen from alcohols, alpha-diols, alkylphenols and esters of fatty acids, these compounds being ethoxylated, propoxylated or glycerolated and having at least one fatty chain comprising, for example, from 8 to 30 carbon atoms, it being possible for the number of ethylene oxide or propylene oxide groups to range from 2 to 50, and for the number of glycerol groups to range from 1 to 30. Maltose derivatives may also be mentioned. Non-limiting mention may also be made of copolymers of ethylene oxide and/or of propylene oxide; condensates of ethylene oxide and/or of propylene oxide with fatty alcohols; polyethoxylated fatty amides comprising, for example, from 2 to 30 mol of ethylene oxide; polyglycerolated fatty amides comprising, for example, from 1.5 to 5 glycerol groups, such as from 1.5 to 4; ethoxylated fatty acid esters of sorbitan comprising from 2 to 30 mol of ethylene oxide; ethoxylated oils of plant origin; fatty acid esters of sucrose; fatty acid esters of polyethylene glycol; polyethoxylated fatty acid mono or diesters of glycerol (C₆-C₂₄)alkylpolyglycosides; N-(C₆-C₂₄)alkylglucamine derivatives; amine oxides such as (C₁₀-C₁₄)alkylamine oxides or N-(C₁₀-C₁₄)acylaminopropylmorpholine oxides; and mixtures thereof.

The nonionic surfactants may preferably be chosen from monooxyalkylenated, polyoxyalkylenated, monoglycerolated or polyglycerolated nonionic surfactants. The oxyalkylene units are more particularly oxyethylene or oxypropylene units, or a combination thereof, and are preferably oxyethylene units.

Examples of monooxyalkylenated or polyoxyalkylenated nonionic surfactants that may be mentioned include:
monooxyalkylenated or polyoxyalkylenated (C₈-C₂₄)alkylphenols,
saturated or unsaturated, linear or branched, monooxyalkylenated or polyoxyalkylenated C₈-C₃₀ alcohols,
saturated or unsaturated, linear or branched, monooxyalkylenated or polyoxyalkylenated C₈-C₃₀ amides,
esters of saturated or unsaturated, linear or branched, C₈-C₃₀ acids and of polyalkylene glycols, monooxyalkylenated or polyoxyalkylenated esters of saturated or unsaturated, linear or branched, C₈-C₃₀ acids and of sorbitol,
saturated or unsaturated, monooxyalkylenated or polyoxyalkylenated plant oils,
condensates of ethylene oxide and/or of propylene oxide, inter alia, alone or as mixtures.

The surfactants preferably contain a number of moles of ethylene oxide and/or of propylene oxide of between 1 and 100 and most preferably between 2 and 50. Advantageously, the nonionic surfactants do not comprise any oxypropylene units.

According to one of the embodiments of the present invention, the polyoxyalkylenated nonionic surfactants are chosen from polyoxyethylenated fatty alcohols (polyethylene glycol ether of fatty alcohol), polyoxyethylenated fatty esters (polyethylene glycol ester of fatty acid), and mixtures of polyoxyethylenated fatty alcohol and polyoxyethylenated fatty ester.

Examples of polyoxyethylenated fatty alcohols (or C₈-C₃₀ alcohols) that may be mentioned include the adducts of ethylene oxide with lauryl alcohol, especially those containing from 2 to 50 oxyethylene units and more particularly those containing from 2 to 20 oxyethylene units (Laureth-2 to Laureth-20, as the CTFA names); the adducts of ethylene oxide with behenyl alcohol, especially those containing from 2 to 50 oxyethylene units and more particularly those containing from 2 to 20 oxyethylene units (Beheneth-2 to Beheneth-20, as the CTFA names); the adducts of ethylene oxide with cetearyl alcohol (mixture of cetyl alcohol and stearyl alcohol), especially those containing from 2 to 30 oxyethylene units (Ceteareth-2 to Ceteareth-30, as the CTFA names); the adducts of ethylene oxide with cetyl alcohol, especially those containing from 2 to 30 oxyethylene units (Ceteth-2 to Ceteth-30, as the CTFA names); the adducts of ethylene oxide with stearyl alcohol, especially those containing from 2 to 50 oxyethylene units and more particularly those containing from 2 to 20 oxyethylene units (Steareth-2 to Steareth-20, as the CTFA names); the adducts of ethylene oxide with isostearyl alcohol, especially those containing from 2 to 50 oxyethylene units (Isosteareth-2 to Isosteareth-50, as the CTFA names); and mixtures thereof.

Examples of polyoxyethylenated fatty esters that may be mentioned include the adducts of ethylene oxide with esters of lauric acid, palmitic acid, stearic acid or behenic acid, and mixtures thereof, especially those containing from 9 to 100 oxyethylene units, such as PEG-9 to PEG-50 laurate (as the CTFA names: PEG-9 laurate to PEG-50 laurate); PEG-9 to PEG-50 palmitate (as the CTFA names: PEG-9 palmitate to PEG-50 palmitate); PEG-9 to PEG-50 stearate (as the CTFA names: PEG-9 stearate to PEG-50 stearate); PEG-9 to PEG-50 palmitostearate; PEG-9 to PEG-50 behenate (as the CTFA names: PEG-9 behenate to PEG-50 behenate); polyethylene glycol 100 EO monostearate (CTFA name: PEG-100 stearate); and mixtures thereof.

According to one preferred embodiment of the present invention, the cosmetic composition which may be used for the present invention comprises at least one polyoxyethylenated fatty alcohol.

According to a more preferred embodiment, the cosmetic composition which may be used for the present invention contains at least one fatty alcohol comprising from 2 to 9 ethyleneoxide units and at least one fatty alcohol comprising from 10 to 30 ethyleneoxide units.

As examples of monoglycerolated or polyglycerolated nonionic surfactants, monoglycerolated or polyglycerolated C₈-C₄₀ alcohols are preferably used.

In particular, the monoglycerolated or polyglycerolated C₈-C₄₀ alcohols correspond to the following formula: 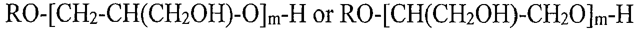 in which R represents a linear or branched C₈-C₄₀ and preferably C₈-C₃₀ alkyl or alkenyl radical, and m represents a number ranging from 1 to 30 and preferably from 1.5 to 10.

As examples of compounds that are suitable in the context of the present invention, mention may be made of lauryl alcohol containing 4 mol of glycerol (INCI name: Polyglyceryl-4 Lauryl Ether), lauryl alcohol containing 1.5 mol of glycerol, oleyl alcohol containing 4 mol of glycerol (INCI name: Polyglyceryl-4 Oleyl Ether), oleyl alcohol containing 2 mol of glycerol (INCI name: Polyglyceryl-2 Oleyl Ether), cetearyl alcohol containing 2 mol of glycerol, cetearyl alcohol containing 6 mol of glycerol, oleocetyl alcohol containing 6 mol of glycerol, and octadecanol containing 6 mol of glycerol.

The alcohol may represent a mixture of alcohols in the same way that the value of m represents a statistical value, which means that, in a commercial product, several species of polyglycerolated fatty alcohols may coexist in the form of a mixture.

Among the monoglycerolated or polyglycerolated alcohols, it is preferable to use C₈/C₁₀ alcohol containing 1 mol of glycerol, C₁₀/C₁₂ alcohol containing 1 mol of glycerol and C₁₂ alcohol containing 1.5 mol of glycerol.

The monoglycerolated or polyglycerolated C₈-C₄₀ fatty esters may correspond to the following formula: 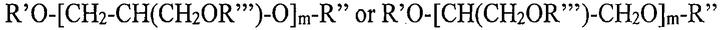 in which each of R', R" and R‴ independently represents a hydrogen atom, or a linear or branched C₈-C₄₀ and preferably C₈-C₃₀ alkyl-CO- or alkenyl-CO-radical, with the proviso that at least one of R', R" and R‴ is not a hydrogen atom, and m represents a number ranging from 1 to 30 and preferably from 1.5 to 10.

Examples of polyoxyethylenated fatty esters that may be mentioned include the adducts of ethylene oxide with esters of lauric acid, palmitic acid, stearic acid or behenic acid, and mixtures thereof, especially those containing from 9 to 100 oxyethylene units, such as PEG-9 to PEG-50 laurate (as the CTFA names: PEG-9 laurate to PEG-50 laurate); PEG-9 to PEG-50 palmitate (as the CTFA names: PEG-9 palmitate to PEG-50 palmitate); PEG-9 to PEG-50 stearate (as the CTFA names: PEG-9 stearate to PEG-50 stearate); PEG-9 to PEG-50 palmitostearate; PEG-9 to PEG-50 behenate (as the CTFA names: PEG-9 behenate to PEG-50 behenate); polyethylene glycol 100 EO monostearate (CTFA name: PEG-100 stearate); and mixtures thereof.

Preferably, the nonionic surfactant may be a nonionic surfactant with an HLB from 8 to 18. HLB is the ratio between the hydrophilic part and the lipophilic part in the molecule. This term HLB is well known to those skilled in the art and is described in "The HLB system. A time-saving guide to emulsifier selection" (published by ICI Americas Inc., 1984).

The amount of the surfactant(s) in the cosmetic composition which may be used for the present invention may be 0.01% by weight or more, preferably 0.05% by weight or more, and more preferably 0.1 % by weight or more, relative to the total weight of the cosmetic composition. The amount of the surfactant(s) in the cosmetic composition which may be used for the present invention may be 10% by weight or less, preferably 8% by weight or less, and more preferably 5% by weight or less, relative to the total weight of the cosmetic composition. The amount of the surfactant(s) in the cosmetic composition which may be used for the present invention may be from 0.01% to 10% by weight, preferably from 0.05% to 8% by weight, and more preferably from 0.1% to 5% by weight, relative to the total weight of the cosmetic composition.

### (Other Ingredients)

The pH of the cosmetic composition which may be used for the present invention may be adjusted to the desired value using acidifying or basifying agents commonly used in cosmetic compositions.

The water phase of the cosmetic composition which may be used for the present invention may preferably be neutral or acidic. Therefore, it is preferable that the pH of the water phase of the cosmetic composition be from 1 to 7, more preferably from 2 to 6, and even more preferably from 3 to 5.

Among the acidifying agents, mention may be made, by way of example, of mineral or organic acids such as hydrochloric acid, ortho-phosphoric acid, sulfuric acid, carboxylic acids such as acetic acid, tartaric acid, citric acid, and lactic acid, and sulfonic acids.

Among the basifying agents, mention may be made, by way of example, of ammonium hydroxide, alkali metal carbonates, alkanolamines such as mono-, di- and triethanolamines and also their derivatives, sodium or potassium hydroxide and compounds of the formula below: 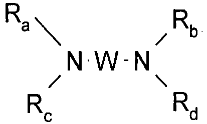 wherein
W denotes an alkylene such as propylene optionally substituted by a hydroxyl or a C₁-C₄ alkyl radical, and Rₐ, R_{b}, R_{c} and R_{d} independently denote a hydrogen atom, an alkyl radical or a C₁-C₄ hydroxyalkyl radical, which may be exemplified by 1,3-propanediamine and derivatives thereof.

The acidifying or basifying agent may be used in an amount ranging from 0.001% to 15% by weight, preferably from 0.01% to 10% by weight, and more preferably from 0.1% to 5% by weight, relative to the total weight of the cosmetic composition.

The cosmetic composition which may be used for the present invention may also contain various adjuvants conventionally used in cosmetic compositions, such as anionic, non-ionic, cationic, and amphoteric or zwitterionic polymers, antioxidants, thickeners, sequestering agents, fragrances, dispersing agents, conditioning agents, film-forming agents, ceramides, preservatives and opacifying agents.

### (Preparation)

The cosmetic composition which may be used for the present invention may be prepared by mixing, for example, the oil(s), water, and other ingredient(s), if necessary, as explained above.

The method and means to mix the above ingredients are not limited. Any conventional method and means can be used to mix the above ingredients to prepare the cosmetic composition which may be used for the present invention.

### [Cosmetic Process]

The cosmetic process according to the present invention comprises the steps of:
attaching at least one microneedle sheet to at least one application surface of at least one applicator
   wherein
   the microneedle sheet comprises a substrate layer and a plurality of microneedles on the substrate layer, the microneedles comprising at least one water-soluble or water-dispersible polymer,
by attaching the substrate layer to the application surface of the applicator, wherein the application surface of the applicator is curved;
   and
applying the applicator onto the keratin substance such that the microneedles contact the keratin substance.

The cosmetic process according to the present invention may be intended for cosmetic treatments of a keratin substance such as the skin or the lips, preferably the skin, and more preferably the skin of the face.

When the microneedle sheet is used, the substrate layer of the microneedle sheet is attached to the application surface of the applicator in order to the microneedle sheet to the applicator.

It is preferable that the microneedle sheet be stored in a container, and taken out from the container when the microneedle sheet is used.

It is preferable that the microneedle sheet comprise at least one adhesive layer, and the adhesive layer be attached to the application surface of the applicator.

It is more preferable that the microneedle sheet comprise at least one releasing layer on the adhesive layer, and that the releasing layer be removed from the surface of the adhesive layer when the microneedle sheet is used.

The applicator with the microneedle sheet is then applied onto the keratin substance by, for example, stamping the application surface onto the keratin substance or rolling the application surface on the keratin substance.

The microneedles of the microneedle sheet penetrate into the keratin substrate to provide cosmetic effects.

The cosmetic process according to the present invention may be used to improve the aesthetic appearance of a keratin substance, for example, by reducing the appearance of wrinkles or by providing the keratin substance with any cosmetic active ingredient, if present, in the microneedles.

A reduction in the amount of matrix, such as epidermis, in the skin tends to lead to a decrease in skin thickness and deterioration of skin elasticity, causing the formation of wrinkles. The microneedles can increase the amount of matrix in the skin thus causing an increase of skin elasticity which results in the reduction of wrinkles on the skin.

If the microneedle, in particular the distal end portion, is swellable, it can swell in the skin to further increase the volume of the microneedle along with its absorption of, for example, water in the skin. Such volume expansion beneath the skin surface of a wrinkle site can effectively push the wrinkles from inside the skin and makes the wrinkles become shallower and wider. Thus, the wrinkles can be reduced or made less noticeable.

Thus, it is preferable that the cosmetic process according to the present invention comprise the step of pressing onto the keratin substance the microneedle sheet to securely insert the microneedles of the microneedle sheet into the keratin substance such as the skin.

In a particular embodiment, the cosmetic process according to the present invention can be used to apply semi-permanent or permanent cosmetic treatments to a keratin substance such as the skin.

### EXAMPLES

The present invention will be described in a more detailed manner by way of examples. However, these examples should not be construed as limiting the scope of the present invention. The examples below are presented as non-limiting illustrations in the field of the present invention.

### [Microneedle Patch]

A microneedle sheet was prepared using sodium hyaluronate with an average molecular weight of 100 K Dalton. The microneedle sheet had a plurality of microneedles on a substrate layer. Each microneedle had the shape of a cone with a length or height of 250 µm and a base diameter of 300 µm. The pitch of the microneedles was 300 µm.

The microneedle sheet was prepared by a standard mold casting process. Sodium hyaluronate, polyvinylpyrrolidone, glycerin, propylene glycol and vitamin C were dissolved in water, and an aqueous solution of these ingredients thus obtained was poured into the cavities of a mold which corresponds to the shape of microneedles and a substrate layer. After drying at room temperature to remove water, microneedles in the cavities were removed from the mold as a microneedle sheet having a plurality of microneedles on a substrate layer.

The microneedle sheet was cut such that it had the shape of a patch to be applied onto an applicator.

The microneedle patch was fixed onto one surface of a flexible PET (polyethylene terephthalate) film, with a pressure-sensitive adhesive. Onto the other surface of the PET film, a pressure-sensitive adhesive comprising a vinyl acetate-acrylate copolymer was applied to form a pressure-sensitive adhesive layer. Onto the free surface of the adhesive layer, a releasing paper was laminated. Thus, a laminated (multi-layered) microneedle patch was prepared.

### [Container]

A tray made from PET was used as a container. A non-woven fabric sheet with a thickness of 2 mm was placed as a porous sheet in the tray. The above microneedle sheet was placed in the tray such that the microneedles faced the porous sheet in the tray. The microneedles of the microneedle sheet were not broken.

### [Microneedle Assembly]

The laminated microneedle patch was attached onto the surface of a cylindrical roller made from stainless steel with a handle made from plastic materials as shown in Figure 3, by peeling off the releasing paper and applying the pressure-sensitive adhesive layer onto the surface of the cylindrical roller, to prepare a microneedle assembly.

### [Evaluation]

Human cadaver skin was excised and trimmed to a thickness of 700 µm using an electric dermatome (Integra Life SciencesTM, Padgett Instruments, Plainsboro, NJ, USA) to prepare a human skin sample.

The roller with the microneedle sheet of the above microneedle assembly was rolled 10 times on the human skin sample, such that the microneedles penetrated into the human skin sample.

Next, the human skin sample was dipped into hot liquid paraffin and cooled to prepare paraffin blocks. The paraffin blocks were cut to prepare 5 human skin cross-sectional slices.

For the human skin sample on which the above microneedle assembly was not rolled, 5 human skin cross-sectional slices were also prepared.

Both sets of human skin cross-sectional slices were then pre-fixed in a Bouin's solution and stained with Lillie-Mayer's Hematoxylin (Muto Pure Chemicals Co., Ltd., Japan) and eosin solution (Wako Pure Chemical Industries). Bright field images of HE-stained sections were observed to confirm the disruption of the skin using a digital camera (DFC295; Leica, Germany) at 200-fold magnification.

Comparing with the cross sectional views of the skin with or without being rolled with the microneedle assembly, it was found that the skin after the rolling treatment with the microneedle assembly showed successful disruptions of the stratum corneum of the skin at some sites of the skin, and that the shape of the disruptions could correspond to the shape of the microneedles.

## Claims

1. A kit comprising:
at least one microneedle sheet (1) comprising a substrate (1-2) layer and a plurality of microneedles (1-1) on the substrate layer, the microneedles comprising at least one water-soluble or water-dispersible polymer;
and
at least one applicator (2) comprising at least one application surface, wherein the application surface of the applicator is curved,
wherein
the substrate layer of the microneedle sheet is capable of being attached to the application surface of the applicator.

2. The kit according to Claim 1, wherein the substrate layer of the microneedle sheet is detachable from the application surface of the applicator, after being attached to the application surface of the applicator.

3. The kit according to Claim 1 or 2, wherein the microneedle sheet comprises at least one base layer, and the base layer is fixed onto the substrate layer.

4. The kit according to Claim 1 or 2, wherein the microneedle sheet comprises at least one adhesive layer, and the adhesive layer is fixed onto the substrate layer.

5. The kit according to Claim 3, wherein the microneedle sheet comprises at least one adhesive layer, and the adhesive layer is fixed onto the base layer.

6. The kit according to Claim 4 or 5, wherein the microneedle sheet comprises at least one releasing layer, and the releasing layer is placed on the adhesive layer.

7. The kit according to any one of Claims 1 to 6, wherein the microneedle has a height of from 50 to 1000 microns, preferably from 100 to 750 microns, and more preferably from 150 to 500 microns.

8. The kit according to any one of Claims 1 to 7, wherein the applicator comprises at least one cylindrical roller having a curved side surface, and the curved side surface of the cylindrical roller comprises the application surface.

9. The kit according to any one of Claims 1 to 8, further comprising at least one container wherein the container is capable of storing the microneedle sheet.

10. The kit according to Claim 9, wherein the container comprises at least one soft and/or porous article, preferably selected from sponges, woven or non-woven fabrics and combinations thereof.

11. The kit according to any one of Claims 1 to 10, further comprising at least one cosmetic composition.

12. A cosmetic process for a keratin substance such as the skin or the lips, comprising the steps of:
attaching at least one microneedle sheet (1) to at least one application surface of at least one applicator (2), wherein the application surface of the applicator is curved, wherein the microneedle sheet comprises a substrate layer (1-2) and a plurality of microneedles (1-1) on the substrate layer, the microneedles comprising at least one water-soluble or water-dispersible polymer, attaching the substrate layer to the application surface of the applicator;
and
applying the applicator onto the keratin substance such that the microneedles contact the keratin substance.

## Patentansprüche

1. Satz, umfassend:
mindestens eine Mikronadelfolie (1), die eine Substratschicht (1-2) und eine Vielzahl von Mikronadeln (1-1) auf der Substratschicht umfasst, wobei die Mikronadeln mindestens ein wasserlösliches oder ein in Wasser dispergierbares Polymer umfassen,
und
mindestens einen Applikator (2), der mindestens eine Auftragefläche umfasst, wobei die Auftragefläche des Applikators gekrümmt ist,
wobei die Substratschicht der Mikronadelfolie an der Auftragefläche des Applikators angebracht werden kann.

2. Satz nach Anspruch 1, wobei die Substratschicht der Mikronadelfolie von der Auftragefläche des Applikators ablösbar ist, nachdem sie an der Auftragefläche des Applikators angebracht worden ist.

3. Satz nach Anspruch 1 oder 2, wobei die Mikronadelfolie mindestens eine Grundschicht umfasst und die Grundschicht auf der Substratschicht befestigt ist.

4. Satz nach Anspruch 1 oder 2, wobei die Mikronadelfolie mindestens eine Klebeschicht umfasst und die Klebeschicht auf der Substratschicht befestigt ist.

5. Satz nach Anspruch 3, wobei die Mikronadelfolie mindestens eine Klebeschicht umfasst und die Klebeschicht auf der Grundschicht befestigt ist.

6. Satz nach Anspruch 4 oder 5, wobei die Mikronadelfolie mindestens eine Abziehschicht umfasst und die Abziehschicht auf der Klebeschicht platziert ist.

7. Satz nach einem der Ansprüche 1 bis 6, wobei die Mikronadel eine Höhe von 50 bis 1000 Mikrometern, vorzugsweise von 100 bis 750 Mikrometern und stärker bevorzugt von 150 bis 500 Mikrometern hat.

8. Satz nach einem der Ansprüche 1 bis 7, wobei der Applikator mindestens eine zylindrische Rolle mit einer gekrümmten Seitenfläche umfasst und die gekrümmte Seitenfläche der zylindrischen Rolle die Auftragefläche umfasst.

9. Satz nach einem der Ansprüche 1 bis 8, ferner umfassend mindestens einen Behälter, wobei die Mikronadelfolie in dem Behälter gelagert werden kann.

10. Satz nach Anspruch 9, wobei der Behälter mindestens einen weichen und/oder porösen Artikel umfasst, der vorzugsweise aus Schwämmen, Geweben oder Vliesen und Kombinationen davon ausgewählt ist.

11. Satz nach einem der Ansprüche 1 bis 10, ferner umfassend mindestens eine kosmetische Zusammensetzung.

12. Kosmetisches Verfahren für eine Keratinsubstanz wie die Haut oder die Lippen, umfassend die folgenden Schritte:
Anbringen mindestens einer Mikronadelfolie (1) an mindestens einer Auftragefläche mindestens eines Applikators (2), wobei die Auftragefläche des Applikators gekrümmt ist,
wobei die Mikronadelfolie eine Substratschicht (1-2) und eine Vielzahl von Mikronadeln (1-1) auf der Substratschicht umfasst, wobei die Mikronadeln mindestens ein wasserlösliches oder ein in Wasser dispergierbares Polymer umfassen,
Anbringen der Substratschicht auf der Auftragefläche des Applikators
und
Aufbringen des Applikators auf die Keratinsubstanz, so dass die Mikronadeln die Keratinsubstanz kontaktieren.

## Revendications

1. Kit comprenant :
au moins une feuille à micro-aiguilles (1) comprenant une couche de substrat (1-2) et une pluralité de micro-aiguilles (1-1) sur la couche de substrat, les micro-aiguilles comprenant au moins un polymère soluble dans l'eau ou dispersible dans l'eau ;
et
au moins un applicateur (2) comprenant au moins une surface d'application, dans lequel la surface d'application de l'applicateur est incurvée,
dans lequel
la couche de substrat de la feuille à micro-aiguilles peut être fixée à la surface d'application de l'applicateur.

2. Kit selon la revendication 1, dans lequel la couche de substrat de la feuille à micro-aiguilles est détachable de la surface d'application de l'applicateur, après avoir été fixée à la surface d'application de l' applicateur.

3. Kit selon la revendication 1 ou 2, dans lequel la feuille à micro-aiguilles comprend au moins une couche de base, et la couche de base est fixée sur la couche de substrat.

4. Kit selon la revendication 1 ou 2, dans lequel la feuille à micro-aiguilles comprend au moins une couche adhésive, et la couche adhésive est fixée sur la couche de substrat.

5. Kit selon la revendication 3, dans lequel la feuille à micro-aiguilles comprend au moins une couche adhésive, et la couche adhésive est fixée sur la couche de base.

6. Kit selon la revendication 4 ou 5, dans lequel la feuille à micro-aiguilles comprend au moins une couche antiadhésive, et la couche antiadhésive est placée sur la couche adhésive.

7. Kit selon l'une quelconque des revendications 1 à 6, dans lequel la micro-aiguille a une hauteur de 50 à 1000 microns, de préférence de 100 à 750 microns, et plus préférablement de 150 à 500 microns.

8. Kit selon l'une quelconque des revendications 1 à 7, dans lequel l'applicateur comprend au moins un rouleau cylindrique ayant une surface latérale incurvée, et la surface latérale incurvée du rouleau cylindrique comprend la surface d'application.

9. Kit selon l'une quelconque des revendications 1 à 8, comprenant en outre au moins un récipient, le récipient étant capable de stocker la feuille à micro-aiguilles.

10. Kit selon la revendication 9, dans lequel le récipient comprend au moins un article souple et/ou poreux, de préférence choisi parmi des éponges, des tissus tissés ou non tissés et des combinaisons de ceux-ci .

11. Kit selon l'une quelconque des revendications 1 à 10, comprenant en outre au moins une composition cosmétique.

12. Procédé cosmétique pour une substance kératinique telle que la peau ou les lèvres, comprenant les étapes de :
fixation d'au moins une feuille à micro-aiguilles (1) à au moins une surface d'application d'au moins un applicateur (2), dans lequel la surface d'application de l'applicateur est incurvée,
dans lequel
la feuille à micro-aiguilles comprend une couche de substrat (1-2) et une pluralité de micro-aiguilles (1-1) sur la couche de substrat, les micro-aiguilles comprenant au moins un polymère soluble dans l'eau ou dispersible dans l'eau,
la fixation de la couche de substrat à la surface d'application de l'applicateur
et
l'application de l'applicateur sur la substance kératinique de sorte que les micro-aiguilles viennent en contact avec la substance kératinique.
